# EUROPEAN PATENT APPLICATION

(11) **EP 1 213 031 A2**
(43) Date of publication of application: **12.06.2002**
(21) Application number: 01310160.5
(22) Date of filing: 05.12.2001
(51) Int. Cl.: A61K 45/06

(54) **Combination treatment for depression, anxiety and psychosis comprising an antidepressant and/or anxiolytic and a D4 receptor antagonist**

(30) Priority: 05.12.2000 US 251284
(71) Applicant: Pfizer Products Inc., Groton, Connecticut 06340 (US)
(72) Inventor: Chappell, Phillip Branch, Groton, Connecticut 06340 (US); Zorn, Stevin Howard, Groton, Connecticut 06340 (US)
(74) Representative: Sewell, Richard Charles

(57) **Abstract**

The present invention relates to a method of treating depression, anxiety or psychosis in a mammal, including a human, by administering to the mammal a D4 receptor antagonist in combination with an antidepressant or an anxiolytic agent. It also relates to pharmaceutical compositions containing a pharmaceutically acceptable carrier, a D4 receptor antagonist and an antidepressant or an anxiolytic agent.

## Description

### Background Of The Invention

The present invention relates to a method of treating depression, anxiety or psychosis in a mammal, including a human, by administering to the mammal a dopamine D4 receptor antagonist (D4 receptor antagonist) in combination with an antidepressant or an anxiolytic agent. It also relates to pharmaceutical compositions containing a pharmaceutically acceptable carrier, a D4 receptor antagonist and an antidepressant or an anxiolytic agent.

### Summary Of The Invention

The present invention relates to a pharmaceutical composition for the treatment of depression, anxiety or psychosis in a mammal, comprising: (a) a compound that exhibits activity as an antidepressant or an antianxiety (*i.e*., anxiolytic) agent, or a pharmaceutically acceptable salt thereof; (b) a D4 receptor antagonist *(i.e.,* dopamine D4 receptor antagonist) or pharmaceutically acceptable salt thereof; and (c) a pharmaceutically acceptable carrier; wherein the active agents "a" and "b" above are present in amounts that render the composition effective in treating, respectively, depression, anxiety or psychosis.

This invention also relates to a method of treating depression, anxiety or psychosis in a mammal, comprising administering to said mammal, respectively, an antidepressant, anxiolytic or antipsychotic effective amount of a pharmaceutical composition comprising: (a) a compound that exhibits activity as, respectively, an antidepressant or an anxiolytic agent, or a pharmaceutically acceptable salt thereof; (b) a D4 receptor antagonist or pharmaceutically acceptable salt thereof; and (c) a pharmaceutically acceptable carrier; wherein the active agents "a" and "b" above are present in amounts that render the composition effective in treating, respectively, depression, anxiety or psychosis.

This invention also relates to a method of treating depression, anxiety or psychosis in a mammal, comprising administering to said mammal: (a) a compound that exhibits activity as, respectively, an antidepressant or an anxiolytic agent, or a pharmaceutically acceptable salt thereof; and (b) a D4 receptor antagonist or pharmaceutically acceptable salt thereof; wherein the active agents "a" and "b" above are present in amounts that render the combination of the two agents effective in treating, respectively, depression, anxiety or psychosis.

It will be appreciated that when using a combination method of the present invention, referred to immediately above, both the D4 receptor antagonist and the antidepressant or antianxiety agent will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the antidepressant or anxiolytic agent may be administered as a tablet and then, within a reasonable period of time, the D4 receptor antagonist may be administered either as an oral dosage form such as a tablet or a fast-dissolving oral dosage form. By a "fast dissolving oral formulation" is meant an oral delivery form which, when placed on the tongue of a patient, dissolves within about seconds.

The compositions of the present invention that contain a D4 receptor antagonist and an antidepressant are useful for the treatment of depression. As used herein, the term "depression" includes depressive disorders, for example, single episodic or recurrent major depressive disorders, dysthymic disorders, cyclothymic disorder, depressive neurosis, and neurotic depression; melancholic depression including anorexia, weight loss, insomnia and early morning waking, and psychomotor retardation; atypical depression (or reactive depression) including increased appetite, hypersomnia, psychomotor agitation or irritability, anxiety and phobias. The term "depression," as used herein, also includes the mood disorders such as mood disorders associated with premenstrual syndrome (PMS) or premenstrual dysphoric disorder (PMDD), seasonal affective disorder and bipolar disorders or manic depression, for example, bipolar I disorder and bipolar II disorder.

Major depression is characterized by feelings of intense sadness and despair, mental slowing and loss of concentration, pessimistic worry, agitation, and self-deprecation. Physical changes also occur, especially in severe or "melancholic" depression. These include insomnia or hypersomnia, anorexia and weight loss (or sometimes overeating), decreased energy and libido, and disruption of normal circadian rhythms of activity, body temperature, and many endocrine functions.

Treatment regimens commonly include the use of tricyclic antidepressants, monoamine oxidase inhibitors, some psychotropic drugs, lithium carbonate, and electroconvulsive therapy (ECT) (see R. J. Baldessarini in *Goodman & Gilman's The Pharmacological Basis of Therapeutics,* 9th Edition, Chapter 19, McGraw-Hill, 1996 for a review). More recently, new classes of antidepressant drugs have been or are being developed including selective serotonin reuptake inhibitors (SSRIs), specific monoamine reuptake inhibitors and 5-HT_{IA/ID} receptor agonists, antagonists and partial agonists.

Other mood disorders encompassed within the term "depression" include dysthymic disorder with early or late onset and with or without atypical features; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

The compositions of the present invention that contain a D4 receptor antagonist and an anxiolytic agent are useful for the treatment of anxiety. As used herein, the term "anxiety" includes anxiety disorders, such as panic disorder with or without agoraphobia, agoraphobia without history of panic disorder, specific phobias, for example, specific animal phobias, social phobias, obsessive-compulsive disorder, stress disorders including post-traumatic stress disorder and acute stress disorder, and generalized anxiety disorders.

Anxiety disorders are generally treated using benzodiazepine sedative-antianxiety agents. Potent benzodiazepines are effective in panic disorder as well as in generalized anxiety disorder, however, the risks associated with drug dependency may limit their long-term use. 5-HT_{IA} receptor partial agonists also have useful anxiolytic and other psychotropic activity, and less likelihood of sedation and dependence (see R. J. Baldessarini in *Goodman & Gilman's The Pharmacological Basis of Therapeutics,* 9th Edition, Chapter 18, McGraw-Hill, 1996 for a review).

"Generalized anxiety" is typically defined as an extended period (*e.g*., at least six months) of excessive anxiety or worry with symptoms on most days of that period. The anxiety and worry is difficult to control and may be accompanied by restlessness, being easily fatigued, difficulty concentrating, irritability, muscle tension, and disturbed sleep.

"Panic disorder" is defined as the presence of recurrent panic attacks followed by at least one month of persistent concern about having another panic attack. A "panic attack" is a discrete period in which there is a sudden onset of intense apprehension, fearfulness or terror. During a panic attack, the individual may experience a variety of symptoms including palpitations, sweating, trembling, shortness of breath, chest pain, nausea and dizziness. Panic disorder may occur with or without agoraphobia.

"Phobias" includes agoraphobia, specific phobias and social phobias. "Agoraphobia" is characterized by an anxiety about being in places or situations from which escape might be difficult or embarrassing or in which help may not be available in the event of a panic attack. Agoraphobia may occur without history of a panic attack. A "specific phobia" is characterized by clinically significant anxiety provoked by a feared object or situation. Specific phobias include the following subtypes: animal type, cued by animals or insects; natural environment type, cued by objects in the natural environment, for example storms, heights or water; blood-injection-injury type, cued by the sight of blood or an injury or by seeing or receiving an injection or other invasive medical procedure; situational type, cued by a specific situation such as public transportation, tunnels, bridges, elevators, flying, driving or enclosed spaces; and other type where fear is cued by other stimuli. Specific phobias may also be referred to as simple phobias. A "social phobia" is characterized by clinically significant anxiety provoked by exposure to certain types of social or performance circumstances. Social phobia may also be referred to as social anxiety disorder.

Other anxiety disorders encompassed within the term "anxiety" include anxiety disorders induced by alcohol, amphetamines, caffeine, cannabis, cocaine, hallucinogens, inhalants, phencyclidine, sedatives, hypnotics, anxiolytics and other substances, and adjustment disorders with anxiety or with mixed anxiety and depression.

Anxiety may be present with or without other disorders such as depression in mixed anxiety and depressive disorders. The compositions of the present invention are therefore useful in the treatment of anxiety with or without accompanying depression.

The compositions of the present invention that contain a D4 receptor antagonist and an antidepressant or anxiolytic agent are useful for the treatment of psychosis. As used herein, the term "psychosis" includes all the specific disorders, including types and subtypes, listed in the DSM-IV™ under the category of schizophrenia and other psychotic disorders. These include schizophrenia, for example, of the paranoid, disorganized, catatonic, undifferentiated, or residual type, schizophreniform disorder, schizoaffective disorder, for example, of the bipolar or depressive type, delusional disorder, for example, of the erotomanic, grandiose, jealous, persecutory, somatic, mixed or unspecified type, brief psychotic disorder, shared psychotic disorder, psychotic disorder due to a general medical condition, for example, of the type with delusions or of the type with hallucinations, substance-induced psychotic disorder, for example, of the type with delusions or of the type with hallucinations, and psychotic disorder not otherwise specified. The meanings attributed to the different types and subtypes of schizophrenia and other psychotic disorders are as stated in the DSM-IV™. (See *Diagnostic and Statistical Manual of Mental Disorders,* Fourth Edition, (DSM-IV™), American Psychiatric Association, 1994, p. 273-315).

Psychosis is characterized by major alterations in mental function, severe disturbances in cognitive and perceptual processes *(e.g.,* hallucinations, delusions), inability to distinguish reality from fantasy, impaired reality testing and disturbances of feeling and behavior. Psychoses may be acute or chronic and functional or organic. They can occur in children, adolescents, adults and the elderly. (See Ayd, Jr., Frank J., *Lexicon of Psychiatry, Neurology and the Neurosciences,* Williams & Wilkins, Baltimore, 1995, p. 543).

Psychotic disorders are generally treated using miscellaneous antipsychotic agents, including Clorazil™, Haldol® , Loxitane® , Moban® , Navane® , Orap® , Risperdal® , Seoquel™ and Zyprex, and phenothiazines and combinations. (See *Physicians' Desk Reference* (PDR® ), 53^{rd} Edition, Medical Economics Company, Inc., 1999, p. 215).

The compositions of the present invention are especially useful for the treatment of depression, anxiety or psychosis, where the use of an antidepressant, anxiolytic agent or antipsychotic agent, respectively, is generally prescribed. By the use of a combination of a D4 receptor antagonist and an antidepressant or anxiolytic agent in accordance with the present invention, it is possible to treat depression and/or anxiety and/or psychosis in patients for whom conventional antidepressant, antianxiety or antipsychotic therapy might not be wholly successful or where dependence upon the antidepressant or antianxiety therapy is prevalent.

Examples of classes of antidepressant agents that may be used in the present invention include norepinephrine reuptake inhibitors, serotonin reuptake inhibitors (SRIs), selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), corticotropin releasing factor (CRF) antagonists, α-adrenoreceptor antagonists, dopamine reuptake inhibitors, NK1 receptor antagonists, 5-HT_{1A/1D} receptor agonists or antagonists and atypical antidepressants.

Another class of antidepressant agents that may be used in the present invention are noradrenergic and specific serotonergic antidepressants (NaSSAs). An example of a NaSSA is mirtazapine.

Examples of norepinephrine reuptake inhibitors that may be used in the present invention include tertiary amine tricyclics and secondary amine tricyclics. Examples of tertiary amine tricyclics include: amitriptyline, clomipramine, doxepin, imipramine and trimipramine, and pharmaceutically acceptable salts thereof. Examples of secondary amine tricyclics include: amoxapine, desipramine, maprotiline, nortriptyline and protriptyline, and pharmaceutically acceptable salts thereof.

Another norepinephrine reuptake inhibitor that may be used in the present invention is reboxetine.

Examples of selective serotonin reuptake inhibitors that may be used in the present invention include: fluoxetine, fluvoxamine, paroxetine and sertraline, and pharmaceutically acceptable salts thereof.

Examples of monoamine oxidase inhibitors that may be used in the present invention include: isocarboxazid, phenelzine, tranylcypromine and selegiline, and pharmaceutically acceptable salts thereof.

Examples of reversible inhibitors of monoamine oxidase that may be used in the present invention include: moclobemide, and pharmaceutically acceptable salts thereof.

Examples of serotonin and noradrenaline reuptake inhibitors that may be used in the present invention include: venlafaxine, and pharmaceutically acceptable salts thereof.

Examples of CRF antagonists that may be used in the present invention include those compounds described in International Patent Application Nos. WO 94/13643, WO 94/13644, WO 94/13661, WO 94/13676 and WO 94/13677.

Examples of dopamine reuptake inhibitors that may be used in the present invention include: methylphenidate, destroamphetamine, bupropion, pemoline, amphetamine and methamphetamine and pharmaceutically acceptable salts thereof, or Adderall® .

Examples of NK1 receptor antagonists that may be used in the methods and pharmaceutical compositions of this invention include the following compounds, and their pharmaceutically acceptable salts, the activity and synthesis of which is referred to in U.S. Patent No. 2,114,848, issued October 7, 1998 and International Patent Application No. WO 93/00331, published January 7, 1993, and U.S. Patent No. 5,744,480, issued April 28, 1998, and EP 0589924, all of which are incorporated herein by reference in their entirety:
(2S,3S)-3-(5-tert-butyl-2-methoxybenzyl)amino-2-(3-trifluoromethoxyphenyl)piperidine;
(2S,3S)-3-(2-isopropoxy-5-trifluoromethoxybenzyl)amino-2-phenyl-piperidine;
(2S,3S)-3-(2-ethoxy-5-trifluoromethoxybenzyl)amino-2-phenyl-piperidine;
(2S,3S)-3-(2-methoxy-5-trifluoromethoxybenzyl)-amino-2-phenylpiperidine;
(2S,3S)-3(-5-tert-butyl-2-trifluoromethoxybenzyl)amino-2-phenylpiperidine;
2-(diphenylmethyl)-N-(2-methoxy-5-trifluoromethoxy-phenyl)methyl-1-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(5-tert-butyl-2-trifluoromethoxybenzyl)amino-2-phenylpiperidine;
(2S,3S)-3-(2-isopropoxy-5-trifluoromethoxybenzyl)amino-2-phenylpiperidine;
(2S,3S)-3-(2-difluoromethoxy-5-trifluoromethoxybenzyl)-amino-2-phenylpiperidine;
(2S,3S)-2-phenyl-3-[2-(2,2,2-trifluoroethoxybenzyl)-aminopiperidine; and
(2S,3S)-2-phenyl-3-(2-trifluoromethoxybenzyl)]aminopiperidine.

Other examples of NK1 receptor antagonists that may be used in the methods and pharmaceutical compositions of this invention include compounds of the formula A, and their pharmaceutically acceptable salts, the activity and synthesis of which is referred to in International Application PCT/IB01/00629, filed April 6, 2001, all of which are incorporated herein by reference in their entirety: 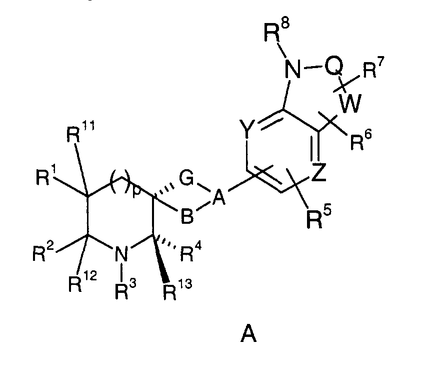 wherein
Q is C=NH, C=CH₂, C=S, C=O, SO or SO₂;
A is CH, CH₂, C(C₁-C₆)alkyl, CH(C₁-C₆)alkyl, C(CF₃) or CH(CF₃), with the proviso that when B is present, A must be either CH, C(C₁-C₆)alkyl or C(CF₃);
B is absent or is methylene or ethylene;
each of Y and Z is N or CH, with the proviso that Y and Z can not both be N;
G is NH(CH₂)_{q}, S(CH₂)_{q} or O(CH₂)_{q}, wherein q is zero or one;
W is a one carbon linking group (i.e., methylene) or a saturated or unsaturated two or three carbon linking group, wherein each of the foregoing W groups can optionally be substituted with one substituent R⁷ or two substituents R⁷ and R⁶, or W is a one carbon linking group that forms, together with a 2, 3, 4 or 5 carbon chain, a 3, 4, 5 or 6 membered spiro ring, respectively;
or W is a saturated two carbon chain linking group that forms, together with a separate 1, 2 or 3 carbon chain, a fused 3, 4 or 5 membered ring, respectively;
or W is a saturated two carbon chain linking group, wherein one of the two carbons in the chain forms, together with a separate 2, 3, 4 or 5 carbon chain, a 3, 4, 5 or 6 membered spiro ring, respectively;
p is zero, one or two;
R³ is selected from hydrogen, COR⁹, CO₂R⁹, optionally substituted phenyl, optionally substituted heterocyclic rings, and optionally substituted (C₁-C₈)alkyl wherein one of the CH₂ groups of said (C₁-C₈) alkyl may optionally be replaced with a sulfur, oxygen or carbonyl group and wherein said (C₁-C₈)alkyl can optionally be substituted with from one to three substituents, preferably with zero substituents or one substituent, independently selected from hydroxy, oxo, phenyl-(C₁-C₃)alkoxy, phenyl, cyano, halo, optionally substituted heterocyclic rings, NR⁹COR¹⁰, NR⁹CO₂R¹⁰, CONR⁹R¹⁰, COR⁹, CO₂R⁹, NR⁹R¹⁰, and (C₁-C₆)alkoxy optionally substituted with from one to seven fluorine atoms, preferably with from zero to three fluorine atoms;
and wherein the heterocyclic rings of R³ and the heterocyclic ring substituents on the alkyl groups of R³ are selected, independently, from 3 to 7 membered saturated or unsaturated monocyclic rings containing from 1 to 4 ring heteroatoms, and 8 to 12 membered saturated or unsaturated bicyclic rings containing from 1 to 4 ring heteroatoms, wherein said heteroatoms are selected, independently, from oxygen, nitrogen and sulfur, with the proviso that there can not be two adjacent ring oxygen atoms or two adjacent ring sulfur atoms in either the monocyclic or bicyclic heterocyclic rings, and with the proviso that heterocyclic rings formed from NR⁹R¹⁰ or CONR⁹R¹⁰ must contain at least one nitrogen atom;
and wherein the heterocyclic rings of R³ and the heterocyclic ring substituents on the alkyl groups of R³ can optionally be substituted with one or more substituents, preferably with zero, one or two substituents, independently selected from oxo, hydroxy, thioxo, halo, cyano, phenyl, (CH₂)ₘNR⁹R¹⁰, NR⁹COR¹⁰, (CH₂)ₘOR⁹, wherein m is zero, one or two, and (C₁-C₆)alkyl optionally substituted with one or more substituents, preferably with from zero to two substituents, independently selected from halo, CF₃, methoxy and phenyl;
and wherein the phenyl groups of R³ and the phenyl substituents in the alkyl groups of R³ can optionally be substituted with one or more substitutents, preferably with from zero to two substituents, independently selected from the group consisting of halo, cyano, nitro, CF₃, (CH₂)ₘNR⁹R¹⁰, wherein m is zero, one or two, NR⁹COR¹⁰, NR⁹CO₂R¹⁰, CONR⁹R¹⁰, CO₂NR⁹R¹⁰, COR⁹, CO₂R⁹, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆)alkenyl optionally substituted with from one to seven fluorine atoms, preferably with from zero to three fluorine atoms;
each of R¹, R², R¹¹, R¹² and R¹³ are selected, independently, from hydrogen and (C₁-C₆)alkyl optionally substituted with one or more substituents, preferably with zero, one or two substituents, that are selected, independently, from hydroxy, oxo, (C₁-C₆)alkoxy and cyano;
or R¹ and R², together with the carbon atoms to which they are attached, or R² and R³, together with the carbon and nitrogen to which they are attached, respectively, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms; or R¹ and R², together with the carbons to which they are attached, form a 5 or 6 membered, saturated or unsaturated carbocyclic ring, and wherein said heterocyclic and carbocyclic rings formed by R¹ and R² or by R² and R³ can be substituted with one or more substituents, preferably with zero substituents or one substituent, independently selected from halo, oxo, NR⁹R¹⁰, (C₁-C₆)alkyl optionally substituted with from one to seven fluorine atoms, preferably with from zero to three fluorine atoms, and (C₁-C₆)alkoxy optionally substituted with from one to seven fluorine atoms, preferably with from zero to three fluorine atoms;
or R¹² and R¹³, together with the carbon atoms to which they are attached, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms, or R¹² and R¹³, together with the carbons to which they are attached, form a 5 or 6 membered, saturated or unsaturated carbocyclic ring, and wherein said heterocyclic and carbocyclic rings formed by R¹² and R¹³ can be substituted with one or more substituents, preferably with zero substituents or one substituent, independently selected from NR⁹R¹⁰, halo, phenyl-S-, phenyl-SO-, phenyl-SO₂-,oxo, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms:
   with the proviso that no more than one of R¹ and R², R² and R³, and R¹² and R¹³ can form a ring;
R⁴ is selected from phenyl, 2-, 3- or 4-pyridyl, 2- or 3-thienyl, and pyrimidyl, wherein R⁴ can be optionally substituted with one or more substituents, preferably with zero or one substituent, selected, independently, from halo, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆) alkenyl optionally substituted with from one to seven fluorine atoms, preferably with from zero to three fluorine atoms;
R⁵ and R⁸ are selected, independently, from hydrogen, -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected, independently, from -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
each R⁹ and each R¹⁰ is selected, independently, from hydrogen, (C₁-C₆)alkyl, hydroxy(C₁-C₆)alkyl, phenyl and CF₃;
or R⁹ and R¹⁰, when R³ is NR⁹R¹⁰ or CONR⁹R¹⁰, can form, together with the nitrogen to which they are attached, an optionally substituted heterocyclic ring that contains at least one nitrogen atom;
and wherein the phenyl groups in the definition of R⁵, R⁶, R⁷ and R⁸ and the phenyl moiety of phenyl (C₁-C₂)alkyl in the definition of R⁵, R⁶, R⁷ and R⁸ can optionally be substituted with one or more substituents, preferably with from zero to two substituents, that are selected, independently, from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms;
with the proviso that: (a) R⁸ can not be halo, hydroxy, cyano, aryloxy, heteroaryloxy, substituted or unsubstituted (C₁-C₆)alkoxy or methyl substituted with from 1-3 fluorine atoms; and (b) when Q is C=O or C=S, and Y and Z are both carbon, and W is a methylene, ethylene or propylene group that is optionally substituted with (C₁-C₆)alkyl or fluoro substituted (C₁-C₆)alkyl, and all of R¹, R², R¹¹, R¹² and R¹³ are hydrogen, and R⁵, R⁶, R⁷, and R⁸ are selected from hydrogen, halo, (C₁-C₆) alkyl optionally substituted with from 1 to 7 fluorine atoms, (C₁-C₆) alkoxy optionally substituted with from 1 to 7 fluorine atoms, then R³ can not be hydrogen.

Examples of compounds of the formula A that are preferred for use in the methods and pharmaceutical compositions of this invention are the following compounds and their pharmaceutically acceptable salts:
7-[(1-Dimethylaminoacetyl-2-phenyl-piperidin-3-ylamino)-methyl]-6-methoxy-1-methyl-3,4-dihydro-1H-quinolin-2-one;
6-Methoxy-1-methyl-7-{[2-phenyl-1-(pyridin-2-yl-acetyl)-piperidin-3-ylamino]-methyl}-3,4-dihydro-1H-quinolin-2-one;
6-Methoxy-1-methyl-7-{[2-phenyl-1-(pyridin-3-yl-acetyl)-piperidin-3-ylamino]-methyl}-3,4-dihydro-1H-quinolin-2-one;
6-Methoxy-1-methyl-7-{[2-phenyl-1-(pyridin-4-yl-acetyl)-piperidin-3-ylamino]-methyl}-3,4-dihydro-1H-quinolin-2-one;
6-Cyclopropoxy-1-methyl-7-[(2-phenyl-piperidin-3-ylamino)-methyl]-3,4-dihydro-1H-quinolin-2-one;
(5-Chloro-2-methoxy-benzyl)-(2-phenyl-octahydro-cyclopenta[b]pyrrol-3-yl)-amine;
6-Methoxy-1-methyl-7-[(1-[1,2,4]oxadiazol-3-ylmethyl-2-phenyl-piperidin-3-ylamino)-methyl]-3,4-dihydro-1H-quinolin-2-one;
7-{[1-(Imidazol-1-yl-acetyl)-2-phenyl-piperidin-3-ylamino]-methyl}-6-methoxy-1-methyl-3,4-dihydro-1H-quinolin-2-one;
1-[3-(2-Methoxy-5-trifluoromethoxy-benzylamino)-2-phenyl-piperidin-1-yl]-2-pyridin-2-yl-ethanone;
1-[3-(2-Methoxy-5-trifluoromethoxy-benzylamino)-2-phenyl- piperidin-1-yl]-2-pyridin-3-yl-ethanone;
1-[3-(2-Methoxy-5-trifluoromethoxy-benzylamino)-2-phenyl-piperidin-1-yl]-2-pyridin-4-yl-ethanone;
2-Imidazol-1-yl-1-[3-(2-methoxy-5-trifluoromethoxy-benzylamino)-2-phenyl-piperidin-1-yl]-ethanone;
2-Dimethylamino-1-[3-(2-methoxy-5-trifluoromethoxy-benzylamino)-2-phenyl-piperidin-1-yl]-ethanone
3-(2-Benzyloxy-5-trifluoromethoxy-phenyl)-6-phenyl-1-oxa-7-aza-spiro[4.5]decane;
1-[3-(2-Methoxy-5-trifluoromethoxy-benzylamino)-2-phenyl-piperidin-1-yl]-2-pyrrolidin-1-yl-ethanone;
(2-Methoxy-5-trifluoromethoxy-benzyl)-(1-[1,2,4]oxadiazol-3-ylmethyl-2-phenyl-piperidin-3-yl)-amine;
7-{[2-(4-Fluoro-phenyl)-piperidin-3-ylamino]-methyl}-6-methoxy-1- methyl-3,4-dihydro-1H-quinolin-2-one;
[1-(2-Imidazol-1-yl-ethyl)-2-phenyl-piperidin-3-yl]-(2-methoxy-5-trifluoromethoxy-benzyl)-amine;
7-{[1-(2-Dimethylam ino-ethyl)-2-phenyl-piperidin-3-ylamino]-methyl}-6-methoxy-1-methyl-3,4-dihydro-1H-quinolin-2-one;
(5-Chloro-2-ethoxy-pyridin-3-ylmethyl)-(2-phenyl-piperidin-3-yl)-amine;
(5-Chloro-2-methoxy-pyridin-3-ylmethyl)-(2-phenyl-piperidin-3-yl)-amine;
Dibenzofuran-2-ylmethyl-(2-phenyl-piperidin-3-yl)-amine;
[3-(Indan-2-yloxy)-4-methoxy-benzyl]-(2-phenyl-piperidin-3-yl)-amine;
6-[(2-Phenyl-piperidin-3-ylamino)-methyl]-chroman-4-one;
(5-Methyl-benzo[b]thiophen-3-ylmethyl)-(2-phenyl-piperidin-3-yl)-amine;
(2,2-Dimethyl-chroman-6-ylmethyl)-(2-phenyl-piperidin-3-yl)-amine;
(1H-Benzoimidazol-5-ylmethyl)-(2-phenyl-piperidin-3-yl)-amine;
1-{2-[(2-Phenyl-piperidin-3-ylamino)-methyl]-phenyl}-pyrrolidin-2-one;
(2-Phenyl-piperidin-3-yl)-[3-(pyridin-2-yloxy)-benzyl]-amine;
[3-(4-Methoxy-phenoxy)-benzyl]-(2-phenyl-piperidin-3-yl)-amine;
(4-Phenoxy-benzyl)-(2-phenyl-piperidin-3-yl)-amine;
(2-Phenyl-piperidin-3-yl)-thiophen-2-ylmethyl-amine;
Furan-2-ylmethyl-(2-phenyl-piperidin-3-yl)-amine;
(5-Methyl-furan-2-ylmethyl)-(2-phenyl-piperidin-3-yl)-amine;
(3-Methyl-thiophen-2-ylmethyl)-(2-phenyl-piperidin-3-yl)-amine;
(2-Phenyl-piperidin-3-yl)-thiophen-3-ylmethyl-amine;
(3-Methyl-benzo[b]thiophen-2-ylmethyl)-(2-phenyl-piperidin-3-yl)-amine;
Benzofuran-2-ylmethyl-(2-phenyl-piperidin-3-yl)-amine;
(5-Ethyl-furan-2-ylmethyl)-(2-phenyl-piperidin-3-yl)-amine;
(5-Chloro-3-methyl-1-phenyl-1H-pyrazol-4-ylmethyl)-(2-phenyl-piperidin-3-yl)-amine;
6-Methoxy-7-{[1-(2-methoxy-ethyl)-2-phenyl-piperidin-3-ylamino]-methyl}-1-methyl-3,4-dihydro-1H-quinolin-2-one;
(5-Methyl-3-phenyl-isoxazol-4-ylmethyl)-(2-phenyl-piperidin-3-yl)-amine;
(3-Phenoxy-benzyl)-(2-phenyl-piperidin-3-yl)-amine;
Furan-3-ylmethyl-(2-phenyl-piperidin-3-yl)-amine;
(3,5-Dimethyl-1-phenyl-1H-pyrazol-4-ylmethyl)-(2-phenyl-piperidin-3-yl)-amine;
(5,7-Dimethoxy-1H-indol-4-ylmethyl)-(2-phenyl-piperidin-3-yl)-amine;
(5-Methoxy-1H-indol-3-ylmethyl)-(2-phenyl-piperidin-3-yl)-amine;
(4-Oxy-quinoxalin-2-ylmethyl)-(2-phenyl-piperidin-3-yl)-amine;
(2-Phenyl-piperidin-3-yl)-quinoxal in-2-ylmethyl-amine;
7-{[1-(2,3-Dihydroxy-propyl)-2-phenyl-piperidin-3-ylamino]-methyl}-6-methoxy-1-methyl-3,4-dihydro-1H-quinolin-2-one;
(2-Methoxy-5-trifluoromethoxy-benzyl)-[2-phenyl-1-(2-pyrrolidin-1-yl-ethyl)-piperidin-3-yl]-amine;
6-Ethoxy-1-methyl-7-[(2-phenyl-piperidin-3-ylamino)-methyl]-3,4-dihydro-1H-quinolin-2-one;
[1-(2-Dimethylamino-ethyl)-2-phenyl-piperidin-3-yl]-(2-methoxy-5-trifluoromethoxy-benzyl)-amine;
3-(2-Cyclopropoxy-5-trifluoromethoxy-phenyl)-6-phenyl-1-oxa-7-aza-spiro[4.5]decane;
[1-(2-Methoxy-ethyl)-2-phenyl-piperidin-3-yl]-(2-methoxy-5-trifluoromethoxy-benzyl)-amine;
6-Hydroxy-1-methyl-7-[(2-phenyl-piperidin-3-ylamino)-methyl]-3,4-dihydro-1H-quinolin-2-one;
6-Methoxy-1-methyl-7-[(2-phenyl-octahydro-cyclopenta[b] pyrrol-3-ylamino)-methyl]-3,4-dihydro-1H-quinolin-2-one;
7-{[2-(4-Fluoro-phenyl)-piperidin-3-ylamino]-methyl}-6-methoxy-3,4-dihydro-1H-quinolin-2-one;
6-Methoxy-1-methyl-7-(6-phenyl-1 -oxa-7-aza-spiro[4.5]dec-3-yl)-3,4-dihydro-1H-quinolin-2-one;
6-Methoxy-1,3,3-trimethyl-5-[(2-phenyl-octahydro-cyclopenta[b]pyrrol-3-ylamino)-methyl]-1,3-dihydro-indol-2-one;
[3-Chloro-2-(4-fluoro-phenoxy)-pyridin-4-ylmethyl]-(2-phenyl-piperidin-3-yl)-amine;
6-Ethoxy-1,3,3-trimethyl-5-[(2-phenyl-piperidin-3-ylamino)-methyl]-1,3-dihydro-indol-2-one;
6-Ethoxy-1,3,3-trimethyl-5-[(2-phenyl-octahydro-cyclopenta[b]pyrrol-3-ylamino)-methyl]-1,3-dihydro-indol-2-one;
6-Isopropoxy-1,3,3-trimethyl-5-[(2-phenyl-piperidin-3-ylamino)-methyl]-1,3-dihydroindol-2-one;
6-Isopropoxy-1,3,3-trimethyl-5-[(2-phenyl-octahydro-cyclopenta[b]pyrrol-3-ylamino)-methyl]-1,3-dihydro-indol-2-one;
6-Ethoxy-1,3,3-trimethyl-5-[(2-phenyl-octahydro-cyclopenta[b]pyrrol-3-ylamino)-methyl]-1,3-dihydro-indol-2-one;
6-Isopropoxy-1,3,3-trimethyl-5-[(2-phenyl-octahydro-cyclopenta[b]pyrrol-3-ylamino)-methyl]-1,3-dihydro-indol-2-one;
7-Isopropoxy-1-methyl-6-[(2-phenyl-piperidin-3-ylamino)-methyl]-3,4-dihydro-1H-quinolin-2-one;
6-Methoxy-1-methyl-7-[(6-methyl-2-phenyl-piperidin-3-ylamino)-methyl]-3,4-dihydro-1H-quinolin-2-one;
6-Methoxy-1,3,3-trimethyl-7-[(2-phenyl-piperidin-3-ylamino)-methyl]-3,4-dihydro-1H-quinolin-2-one;
6-Methoxy-1,3-dimethyl-7-[(2-phenyl-piperidin-3-ylamino)-methyl]-3,4-dihydro-1H-quinolin-2-one;
6-Methoxy-1,3-dimethyl-5-[(2-phenyl-piperidin-3-ylamino)-methyl]-1,3-dihydro-indol-2-one;
6-Methoxy-1-methyl-5-[(2-phenyl-piperidin-3-ylamino)-methyl]-1,3-dihydro-indol-2-one;
5-[(1-Isopropyl-2-phenyl-piperidin-3-ylamino)-methyl]-6-methoxy-1,3,3-trimethyl-1,3-dihydro-indol-2-one;
6-Methoxy-1-methyl-7-[(2-phenyl-1-propyl-piperidin-3-ylamino)-methyl]-3,4-dihydro-1H-quinolin-2-one;
6-Methoxy-1-methyl-7-{[1-(5-methyl-3H-imidazol-4-ylmethyl)-2-phenyl-piperidin-3-ylamino]-methyl}-3,4-dihydro-1H-quinolin-2-one;
7-{[1-(1H-Imidazol-4-ylmethyl)-2-phenyl-piperidin-3-ylamino]-methyl}-6-methoxy-1-methyl-3,4-dihydro-1H-quinolin-2-one;
7-[(1-Isopropyl-2-phenyl-piperidin-3-ylamino)-methyl]-6-methoxy-1-methyl-3,4-dihydro-1H-quinolin-2-one;
6-Methoxy-1,3-dimethyl-7-[(1-methyl-2-phenyl-piperidin-3-ylamino)-methyl]-3,4-dihydro-1H-quinolin-2-one;
5-[(1-Isopropyl-2-phenyl-piperidin-3-ylamino)-methyl]-6-methoxy-1,3,3-trimethyl-1,3-dihydro-indol-2-one
6-Methoxy-1-methyl-7-{[1-(5-oxo-2,5-dihydro-1H-[1,2,4]triazol-3-ylmethyl)-2-phenyl-piperidin-3-ylamino]-methyl}-3,4-dihydro-1H-quinolin-2-one;
6-Methoxy-7-[(2-phenyl-piperidin-3-ylamino)-methyl]-3,4-dihydro-1H-quinolin-2-one;
1-Ethyl-6-methoxy-7-[(2-phenyl-piperidin-3-ylamino)-methyl]-3,4-dihydro-1H-quinolin 2-one;
1-Methanesulfonyl-6-methoxy-7-[(2-phenyl-piperidin-3-ylamino)-methyl]-3,4-dihydro-1H-quinolin-2-one;
6-Methoxy-1,4,4-trimethyl-7-[(2-phenyl-piperidin-3-ylamino)-methyl]-3,4-dihydro-1H-quinolin-2-one;
8-Fluoro-6-methoxy-1,4,4-trimethyl-7-[(2-phenyl-piperidin-3-ylamino)-methyl]-3,4-dihydro-1H-quinolin-2-one;
6-Methoxy-1-methyl-7-[(2-phenyl-piperidin-3-ylamino)-methyl]-3,4-dihydro-1H-quinolin-2-one;
6-Methoxy-1,4-dimethyl-7-[(2-phenyl-piperidin-3-ylamino)-methyl]-3,4-dihydro-1H-quinolin-2-one;
6-Methoxy-2-methyl-7-[(2-phenyl-piperidin-3-ylamino)-methyl]-3,4-dihydro-2H-isoquinolin-1-one;
6-Methoxy-3-methyl-5-[(2-phenyl-piperidin-3-ylamino)-methyl]-1,1a,3,7b-tetrahydro-3-aza-cyclopropa[a]naphthalen-2-one;
6-Methoxy-1-methyl-,3,3-cyclopropyl-5-[(2-phenyl-piperidin-3-ylamino)-methyl]-1,3-dihydro-indol-2-one;
5-Methoxy-1-methyl-,3,3-cyclopropyl-6-[(2-phenyl-piperid in-3-ylamino)-methyl]-1,3-dihydro-indol-2-one;
6-Methoxy-1-methyl-(6-phenyl-1,7-diaza-spiro[4.5]dec-3-yl)-3,4-dihydro-1H-quinolin-2-one;
6-Methoxy-1-methyl-7-phenyl-1,7-diaza-spiro[4.5]dec-3-yl)-3,4-dihydro-1H-quinolin-2-one;
6-Methoxy-3-methyl-5-[(1-phenyl-8-aza-bicyclo[3.2.1]oct-2-ylamino)-methyl]-1,1 a,3,7b- tetrahydro-3-aza-cyclopropa[a]naphthalen-2-one;
(6-Methoxy-1-methyl-2,2-dioxo-1,2,3,4-tetrahydro-2-thiobenzo[c [1,2]thiazin-7-yl-methyl)- (2-phenyl-piperidin-3-yl)-amine;
6-Methoxy-3-methyl-5-[(6-methyl-2-phenyl-piperidin-3-ylamino)-methyl]-1,1a,3,7b-tetrahydro-3-aza-cyclopropa[a]naphthalen-2-one;
6-Methoxy-1-methyl-7-(6-phenyl-1,7-diaza-spiro[4.5]dec-3-yl)-3,4-dihydro-1H-quinolin-2-one;
6-Methoxy-1,3,3-trimethyl-5-[(2-phenyl-piperidin-3-ylamino)-methyl]-1,3-dihydro-pyrrolo[2,3-b]pyridin-2-one;
5-Methoxy-1,3,3-trimethyl-6-[(2-phenyl-piperidin-3-ylamino)-methyl]-1,3-dihydro-pyrrolo[3,2-b]pyridin-2-one;
6-Methoxy-1-methyl-7-[(2-phenyl-piperidin-3-ylamino)-methyl]-3,4-dihydro-1H-[1,5]naphthyridin-2-one;
7-[(6-Ethyl-2-phenyl-piperidin-3-ylamino)-methyl]-6-methoxy-1-methyl-3,4-dihydro-1H-quinolin-2-one;
5-[(6-Ethyl-2-phenyl-piperidin-3-ylamino)-methyl]-6-methoxy-1,3,3-trimethyl-1,3,-dihydro-indol-2-one;
6-Methoxy-1,3,3-trimethyl-5-[(2-phenyl-piperidin-3-ylamino)-methyl]-1,3-dihydro-pyrrolo[2,3-b]pyridin-2-one;
5-Methoxy-1,3,3-trimethyl-6-[(2-phenyl-piperidin-3-ylamino)-methyl]-1,3-dihydro-pyrrolo[3,2-b]pyridin-2-one;
6-Methoxy-1-methyl-7-[(2-phenyl-piperidin-3-ylamino)-methyl]-3,4-dihydro-1H-[1,5]naphthyridin-2-one;
6-Methoxy-3-methyl-5-[(6-methyl-2-phenyl-piperidin-3-ylamino)-methyl]-1,1a,3,7b-tetrahydro-3-aza-cyclopropa[a]naphthalen-2-one; and
6-Methoxy-1-methyl-7-(6-phenyl-1,7-diaza-spiro[4.5]dec-3-yl)-3,4-dihydro-1H-quinolin-2-one.

Other examples of NK1 receptor antagonists that may be used in the methods and pharmaceutical compositions of this invention include the compounds disclosed in European Patent Application No. 01307657.5, and their pharmaceutically acceptable salts, the activity and synthesis of which is referred to in the aforementioned patent application, which application is incorporated herein by reference in its entirety.

Examples of atypical antidepressants that may be used in the present invention include: bupropion, lithium, nefazodone, trazodone and viloxazine, and pharmaceutically acceptable salts thereof. Another suitable atypical antidepressant is sibutramine.

Other antidepressants that may be used in the present invention include adinazolam, alaproclate, amineptine, amitriptyline/chlordiazepoxide combination, atipamezole, azamianserin, bazinaprine, befuraline, bifemelane, binodaline, bipenamol, brofaromine bupropion, caroxazone, cericlamine, cianopramine, cimoxatone, citalopram, clemeprol, clovoxamine, dazepinil, deanol, demexiptiline, dibenzepin, dothiepin, droxidopa, enefexine, estazolam, etoperidone, femoxetine, fengabine, fezolamine, fluotracen, idazoxan, indalpine, indeloxazine, iprindole, levoprotiline, litoxetine, lofepramine, medifoxamine, metapramine, metralindole, mianserin, milnacipran, minaprine, mirtazapine, montirelin, nebracetam, nefopam, nialamide, nomifensine, norfluoxetine, orotirelin, oxaflozane, pinazepam, pirlindone, pizotyline, ritanserin, rolipram, sercloremine, setiptiline, sibutramine, sulbutiamine, sulpiride, teniloxazine, thozalinone, thymoliberin, tianeptine, tiflucarbine, tofenacin, tofisopam, toloxatone, tomoxetine, veralipride, viqualine, zimelidine and zometrapine, and pharmaceutically acceptable salts thereof, and St. John's wort herb, or *Hypericuin* perforatum, or extracts thereof.

Examples of classes of antianxiety agents that may be used in the present invention include benzodiazepines and 5-HT_{IA/1D} agonists or antagonists, especially 5-HT_{IA} partial agonists and 5-HT_{1D} antagonists, corticotropin releasing factor (CRF) antagonists, serotonin reuptake inhibitors (SRls) and GABA receptor agonists. In addition to benzodiazepines, other suitable classes of antianxiety agents are nonbenzodiazepine sedative-hypnotic drugs such as zolpidem; mood-stabilizing drugs such as clobazam, gabapentin, lamotrigine, loreclezole, oxcarbamazepine, stiripentol and vigabatrin; and barbiturates.

Examples of benzodiazepines that may be used in the present invention include: alprazolam, chlordiazepoxide, clonazepam, chlorazepate, diazepam, halazepam, lorazepam, oxazepam and prazepam, and pharmaceutically acceptable salts thereof.

Examples of 5-HT_{1A} receptor agonists or antagonists that may be used in the present invention include, in particular, the 5-HT_{IA} receptor partial agonists buspirone, flesinoxan, gepirone and ipsapirone, and pharmaceutically acceptable salts thereof. An example of a compound with 5-HT_{IA} receptor antagonist/partial agonist activity is pindolol.

Examples of CRF antagonists that may be used in the present invention include those compounds described in International Patent Application Nos. WO 94/13643, WO 94/13644, WO 94/13661, WO 94/13676 and WO 94/13677.

Another class of antianxiety agents that may be used in the present invention are compounds having muscarinic cholinergic activity. Examples of compounds in this class include m1 muscarinic cholinergic receptor agonists such as those compounds described in European Patent Application Nos. 0 709 093, 0 709 094 and 0 773 021, and International Patent Application No. WO 96/12711.

Another class of antianxiety agents that may be used in the present invention are compounds acting on ion channels. Examples of compounds in this class include carbamazepine, lamotrigine and valproate, and pharmaceutically acceptable salts thereof.

Other antidepressants and antianxiety agents that may be used in the present invention include gabapentin, and pharmaceutically acceptable salts thereof.

Other antidepressants and antianxiety agents that may be used in the methods and pharmaceutical compositions of this invention include compounds of the formula B, which compounds act as 5-HT_{1A/1D} agonists and antagonists, and their pharmaceutically acceptable salts, the activity and synthesis of which is referred to in European Patent Application EP0929528 and International Patent Application No. WO 98/14433, published April 9, 1998, all of which are incorporated herein by reference in their entirety: 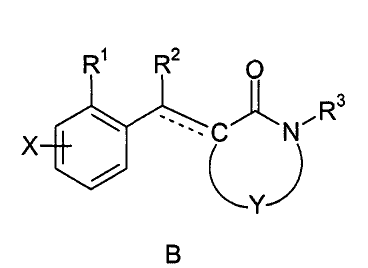 wherein R¹ is a group of the formula G¹, G², G³, G⁴, G⁵, G⁶ or G⁷ depicted below, 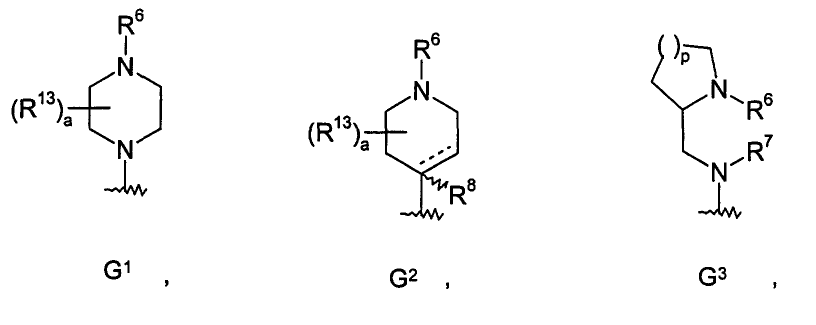 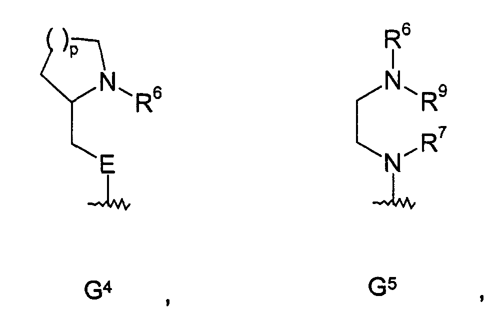 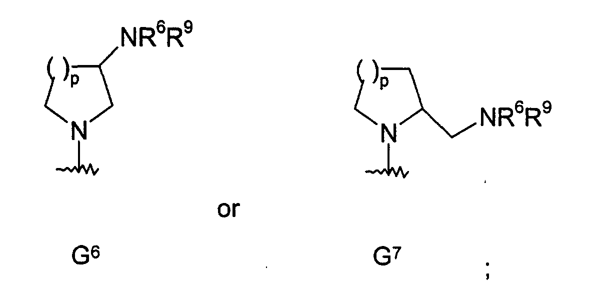 a is zero to eight;
each R¹³ is, independently, (C₁-C₄)alkyl or a (C₁-C₄)methylene bridge from one of the ring carbons of the piperazine or piperidine ring of G¹ or G², respectively, to the same or another ring carbon or a ring nitrogen of the piperazine or piperidine ring of G¹ or G², respectively, having an available bonding site, or to a ring carbon of R⁶ having an available bonding site;
E is oxygen, sulfur, SO or SO₂;
X is hydrogen, chloro, fluoro, bromo, iodo, cyano, (C₁-C₆)alkyl, hydroxy, trifluoromethyl, (C₁-C₆)alkoxy, -SOₜ(C₁-C₆)alkyl wherein t is zero, one or two, -CO₂R¹⁰ or -CONR¹¹R¹²;
Y is an optionally substituted (C₁-C₄) heteroalkyl bridge that, together with the atoms to which it is attached, forms a five to seven membered heterocycle containing two to four heteroatoms selected from the group consisting of 1,3-oxazolidin-4-on-5-yl, 1,3-oxazolidin-2,4-dion-5-yl, 4,5-dihydro-1,2-oxazolidin-3-on-4-yl, 1,3-thiazolidin-4-on-5-yl, 1,3-thiazolidin-2,4-dion-5-yl, 1,3-pyrazolidin-4-on-5-yl, 1,3-imidazolidin-2,4-dion-5-yl, 1,2-pyrazolidin-3-on-4-yl, 1,2-thiazolidin-1,1,3-trion-4-yl, 1,2-thiazolidin-3-on-4-yl, tetrahydro-1,2-oxazin-3-on-4-yl, tetrahydro-1,3-oxazin-4-on-5-yl, tetrahydro-1,3-oxazin-2,4-dion-5-yl, morpholin-3-on-2-yl, morpholin-3,5-dion-2-yl, 2,3-dihydro-1,4-oxazin-3-on-2-yl, tetrahydro-1,3-thiazin-4-on-5-yl, tetrahydro-1,3-thiazin-2,4-dion-5-yl, tetrahydro-1,2-thiazin-3-on-4-yl, thiomorpholin-3-on-2-yl, thiomorpholin-3,5-dion-2-yl, 2,3-dihydro-1,4-thiazin-3-on-2-yl, hexahydro-1,2-diazin-3-on-4-yl, 4,5-dihydro-2H-pyridazin-3-on-4-yl, hexahydro-1,3-diazin-4-on-5-yl, hexahydro-1,3-diazin-2,4-dion-5-yl, piperazin-2-on-3-yl, piperazin-2,6-dion-3-yl, tetrahydro-1,3,4-thiadiazin-5-on-6-yl, 5,6-dihydro-1,3,4-thiadiazin-5-on-6-yl, 1,3,4-oxadiazin-5-on-6-yl, 5,6-dihydro-1,2,4-oxadiazin-5-on-6-yl, tetrahydro-1,2,4-oxadiazin-5-on-6-yl, 1,2,4-triazin-5-on-6-yl, tetrahydro-1,2,4-oxadiazin-5-on-6-yl, 5,6-dihydro-1-2,4-oxadiazin-5-on-6-yl, 1,2,4-oxadiazin-3,5-dion-6-yl, 1,2,4-trazin-6-on-5-yl, hexahydro-1,2-oxazepin-3-on-2-yl, hexahydro-1,3-oxazepin-4-on-5-yl, hexahydro-1,4-oxazepin-3-on-2-yl, hexahydro-1,4-oxazepin-3,5-dion-2-yl, hexahydro-1,4-oxazepin-3,5-dion-6-yl, 2,3,5,6-tetrahydro-1-4-oxazepin-5,7-dion-6-yl, hexahydro-1,4-oxazepin-5-on-6-yl, hexahydro-1,3-oxazepin-2,4-dion-5-yl, hexahydro-1,2-thiazepin-3-on-4-yl, hexahydro-1,4-thiazepin-3-on-2-yl, 2,3,4,5-tetrahydro-1,4-thiazepin-3-on-2-yl, hexahydro-1,4-thiazepin-3,5-dion-2-yl, hexahydro-1,4-thiazepin-3,5-dion-6-yl, 2,3,6,7-tetrahydro-1,4-thiazepin-5-on-6-yl, 6,7-dihydro-1,4-thiazepin-5-on-6-yl, hexahydro-1,3-thiazepin-2,4-dion-5-yl, hexahydro-1,2-diazepin-3-on-4-yl, hexahydro-1,3-diazepin-2,4-dion-5-yl, hexahydro-1,4-diazepin-2-on-3-yl, hexahydro-1,4-diazepin-5-on-6-yl, hexahydro-1,4-diazepin-5,7-dion-6-yl, hexahydro-1,3,5-thiadiazepin-3-on-7-yl, 4,5,6,7-tetrahydro-1-3,5-thiadiazepin-6-on-7-yl, and 2,3,5,6-tetrahydro-1,2,4-triazepin-3,5-dion-7-yl; wherein the substituents on any of the carbon atoms capable of supporting an additional bond, of said (C₁-C₄) heteroalkyl bridge, are chloro, fluoro, (C₁-C₆)alkyl, (C₁-C₆)alkoxy, trifluoromethyl or cyano; wherein the substituents on any of the nitrogen atoms capable of supporting an additional bond, of said (C₁-C₄) heteroalkyl bridge, are (C₁-C₆)alkyl or trifluoromethyl;
R² is hydrogen, (C₁-C₄)alkyl, phenyl or naphthyl, wherein said phenyl or naphthyl may optionally be substituted with one or more substituents independently selected from chloro, fluoro, bromo, iodo, (C₁-C₆)alkyl, (C₁-C₆)alkoxy, trifluoromethyl, cyano and -SOₖ(C₁-C₆)alkyl wherein k is zero, one or two;
R³ is -(CH₂)ₘB, wherein m is zero, one, two or three and B is hydrogen, phenyl, naphthyl or a 5 or 6 membered heteroaryl group containing from one to four heteroatoms in the ring, and wherein each of the foregoing phenyl, naphthyl and heteroaryl groups may optionally be substituted with one or more substituents independently selected from chloro, fluoro, bromo, iodo, (C₁-C₆)alkyl, (C₁-C₆)alkoxy, (C₁-C₆) alkoxy-(C₁-C₆)alkyl-, trifluoromethyl, trifluoromethoxy, cyano, hydroxy, -COOH and -SOₙ(C₁-C₆)alkyl wherein n is zero, one or two;
R⁶ is selected from the group consisting of hydrogen, (C₁-C₆)alkyl optionally substituted with (C₁-C₆)alkoxy or one to three fluorine atoms, or [(C₁-C₄)alkyl]aryl wherein the aryl moiety is phenyl, naphthyl, or heteroaryl-(CH₂)_{q}-, wherein the heteroaryl moiety is selected from the group consisting of pyridyl, pyrimidyl, benzoxazolyl, benzothiazolyl, benzisoxazolyl and benzisothiazolyl and q is zero, one, two, three or four, and wherein said aryl and heteroaryl moieties may optionally be substituted with one or more substituents independently selected from the group consisting of chloro, fluoro, bromo, iodo, (C₁-C₆)alkyl, (C₁-C₆)alkoxy, trifluoromethyl, cyano and -SO_{g}(C₁-C₆)alkyl, wherein g is zero, one or two;
R⁷ is selected from the group consisting of hydrogen, (C₁-C₆)alkyl, [(C₁-C₄)alkyl]aryl wherein the aryl moiety is phenyl, naphthyl, or heteroaryl-(CH₂)ᵣ-, wherein the heteroaryl moiety is selected from the group consisting of pyridyl, pyrimidyl, benzoxazolyl, benzothiazolyl, benzisoxazolyl and benzisothiazolyl and r is zero, one, two, three or four, and wherein said aryl and heteroaryl moieties may optionally be substituted with one or more substituents independently selected from the group consisting of chloro, fluoro, bromo, iodo, (C₁-C₆)alkyl, (C₁-C₆)alkoxy, trifluoromethyl, -C(=O)-(C₁-C₆)alkyl, cyano and -SOⱼ(C₁-C₆)alkyl, wherein j is zero, one or two;
or R⁶ and R⁷ taken together form a 2 to 4 carbon chain;
R⁸ is hydrogen or (C₁-C₃)alkyl;
R⁹ is hydrogen or (C₁-C₆)alkyl;
or R⁶ and R⁹, together with the nitrogen atom to which they are attached, form a 5 to 7 membered heteroalkyl ring that may contain from zero to four heteroatoms selected from nitrogen, sulfur and oxygen;
and p is one, two, or three;
each of R¹⁰, R¹¹ and R¹² is selected, independently, from the radicals set forth in the definition of R²; or R¹¹ and R¹², together with the nitrogen to which they are attached, form a 5 to 7 membered heteroalkyl ring that may contain from zero to four heteroatoms selected from nitrogen, sulfur and oxygen; and
the broken lines indicate optional double bonds, with the proviso that when the broken line in G² is a double bond that R⁸ is absent.

Other antidepressants and antianxiety agents that may be used in the methods and pharmaceutical compositions of this invention include compounds of the formula C, which compounds act as monoamine reuptake inhibitors, and their pharmaceutically acceptable salts, the activity and synthesis of which is referred to in U.S. Patent Application No. 09/529,207, filed February 2, 2000 and International Patent Application No. WO 00/50380, published August 31, 2000, all of which are incorporated herein by reference in their entirety: 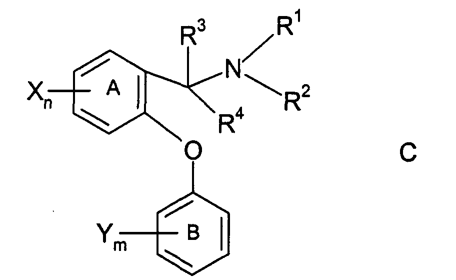
wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl; and
each X and each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl wherein R⁵ is hydrogen or (C₁-C₆)alkyl, and SOₚ(C₁-C₆)alkyl wherein p is zero, one or two;
with the proviso that: (a) no more than one of NR¹R², CR³R⁴ and R²NCR³ can form a ring; and (b) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups.

Examples of D4 receptor antagonists that may be used in the methods and pharmaceutical compositions of this invention are compounds of the formula 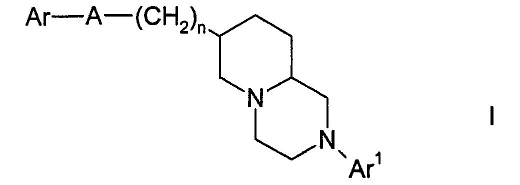 and their pharmaceutically acceptable salts, wherein Ar is phenyl, naphthyl, benzoxazolonyl, indolyl, indolonyl, benzimidazolyl, quinolyl, furyl, benzofuryl, thienyl, benzothienyl, oxazolyl or benzoxazolyl;
Ar¹ is phenyl, pyridinyl, pyridazinyl, pyrimidinyl or pyrazinyl;
A is O, S, SO, SO₂, C=O, CHOH or -(CR³R⁴)-;
n is 0, 1 or 2;
each of Ar and Ar¹ may be independently and optionally substituted with one to four substituents independently selected from the group consisting of fluoro, chloro, bromo, iodo, cyano, nitro, thiocyano, -SR, -SOR, -SO₂R, -NHSO₂R, -(C₁-C₆)alkoxy, -NR¹R², -NRCOR¹, -CONR¹R², phenyl, -COR, -COOR, -(C₁-C₆)alkyl, -(C₁-C₆)alkyl substituted with one to six halogens independently selected from fluoro, chloro, bromo and iodo, -(C₃-C₆)cycloalkyl and trifluoromethoxy;
each and every R, R¹, and R² is independently selected from the group consisting of hydrogen, -(C₁-C₆)alkyl, -(C₁-C₆)alkyl substituted with one to thirteen halogens independently selected from fluoro, chloro, bromo and iodo, phenyl, benzyl, -(C₂-C₆)alkenyl, -(C₃-C₆)cycloalkyl and -(C₁-C₆)alkoxy; and
each and every R³ and R⁴ is independently selected from the group consisting of hydrogen, methyl, ethyl, n-propyl and i-propyl.

In certain embodiments, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula I, wherein Ar is phenyl, naphthyl, benzoxazolonyl, indolyl, indolonyl, benzimidazolyl or quinolyl; A is O, S, SO₂, C=O, CHOH or CH₂; n is 0 or 1; wherein Ar and Ar¹ may be independently substituted with up to three substituents independently selected from the group consisting of fluoro, chloro, cyano, -NR¹R², -(C₁-C₆)alkoxy, -COOR, -CONR¹R² and -(C₁-C₆)alkyl; and the pharmaceutically acceptable salts thereof.

In other embodiments, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula I, wherein A is O or S; n is 1; Ar is phenyl or substituted phenyl; and the pharmaceutically acceptable salts thereof; or A is CH₂; n is 0; Ar is benzoxazolonyl or substituted benzoxazolonyl; and the pharmaceutically acceptable salts thereof; or wherein A is CH₂; n is 0; Ar is indolyl or substituted indolyl; and the pharmaceutically acceptable salts thereof; or wherein A is C=O or CHOH; n is 0 or 1; Ar is phenyl or substituted phenyl; and the pharmaceutically acceptable salts thereof; or wherein A is O; Ar is fluorophenyl, difluorophenyl or cyanophenyl; Ar¹ is chloropyridinyl; and the pharmaceutically acceptable salts thereof; or wherein A is O; Ar is fluorophenyl, difluorophenyl or cyanophenyl; Ar¹ is fluoropyrimidinyl; and the pharmaceutically acceptable salts thereof; or wherein A is O; Ar is fluorophenyl, difluorophenyl or cyanophenyl; Ar¹ is fluorophenyl; and the pharmaceutically acceptable salts thereof; or wherein Ar¹ is 5-chloro-pyridin-2-yl; and the pharmaceutically acceptable salts thereof; or wherein Ar¹ is 5-fluoro-pyrimidin-2-yl; and the pharmaceutically acceptable salts thereof.

In a preferred aspect of the invention, A is O.

In another aspect, A is S, SO, or SO₂.

In another aspect, A is C=O or CHOH.

In another preferred aspect, A is CH₂.

In another preferred aspect, Ar is phenyl or substituted phenyl.

In another preferred aspect, Ar is naphthyl or substituted naphthyl.

In another preferred aspect, Ar is benzoxazolonyl or substituted benzoxazolonyl.

In another preferred aspect, Ar is indolyl or substituted indolyl.

In another preferred aspect, Ar is indolonyl or substituted indolonyl.

In another preferred aspect, Ar is benzimidazolyl or substituted benzimidazolyl.

In another preferred aspect, Ar is quinolyl or substituted quinolyl.

In another preferred aspect, Ar¹ is phenyl or substituted phenyl.

In another preferred aspect, Ar¹ is pyridinyl or substituted pyridinyl.

In another preferred aspect, Ar¹ is pyridazinyl or substituted pyridazinyl.

In another preferred aspect, Ar¹ is pyrimidinyl or substituted pyrimidinyl.

In another preferred aspect, Ar¹ is pyrazinyl or substituted pyrazinyl.

Preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formula I and their pharmaceutically acceptable salts:
(7R,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7R,9aS)-7-(3,5-difluorophenoxy)methyl-2-(5-chloro-pyridin-2-yl)-2,3,4,6,7,8,9,9a-octahydro-1*H-*pyrido[1,2-a]pyrazine;
3-[(7R,9aS)-2-(5-chloro-pyridin-2-yl)-2,3,4,6,7,8,9,9a-octahydro-1*H*-pyrido[1,2-a]pyrazin-7-ylmethyl]-3*H*-benzooxazol-2-one;
3-[(7R,9aS)-2-(5-fluoro-pyrimidin-2-yl)-2,3,4,6,7,8,9,9a-octahydro-1*H*-pyrido[1,2-a]pyrazin-7-ylmethyl]-3*H*-benzoxazol-2-one;
(7R,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,5-difluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,4-difluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-iodophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-fluorophenoxy)methyl-2-(4-fluorophenyl)-2,3,4,6,7,8,9,9a-octahydro-1*H*-pyrido[1,2-a]pyrazine;
(7S,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2-carbomethoxy-4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2-bromo-4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(4-fluoro-2-trifluoromethylphenoxy)methyl-2-(5-fluoro-pyrimidin-2-yl)-2,3,4,6,7,8,9,9a-octahydro-1*H-*pyrido[1,2-a]pyrazine;
(7S,9aS)-7-(3,5-difluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluoro-2-methylphenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2,4-difluorophenoxy)methyl-2-(5-fluoro-pyrimidin-2-yl)-2,3,4,6,7,8,9,9a-octahydro-1*H*-pyrido[1,2-a]pyrazine;
(75,9aS)-7-(3-methyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,4-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,5-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-cyano-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-trifluoromethyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(4-trifluoromethyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-trifluoromethoxy-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-methoxy-phenoxy)methyl-2-(5-fluoropyrimidin-2-yl)-2,3,4,6,7,8,9,9a-octahydro-1*H*-pyrido[1,2-a]pyrazine; and
(7S,9aS)-7-(4-methoxy-phenoxy)methyl-2-(5-fluoropyrimidin-2-yl)-2,3,4,6,7,8,9,9a-octahydro-1*H*-pyrido[1,2-a]pyrazine.

In the compounds of formula I, the term "one or more substituents", includes from one to the maximum number of substituents possible based on the number of available bonding sites.

Unless otherwise indicated, the term "alkyl", as used in the compounds of formula I, includes saturated monovalent hydrocarbon radicals having straight, branched or cyclic moieties or combinations thereof.

Unless otherwise indicated, the term "alkoxy", as used in the compounds of formula I, refers to radicals having the formula -O-alkyl, wherein "alkyl" is defined as above for the compounds of formula I.

Unless otherwise indicated, the term "halo" or "halogen", as used in the compounds of formula I, includes fluoro, chloro, bromo and iodo.

Other examples of D4 receptor antagonists that may be used in the methods and pharmaceutical compositions of this invention are compounds of the formula 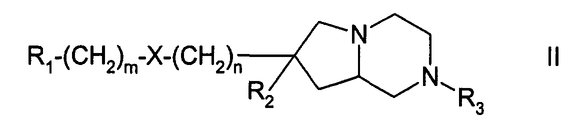 and their pharmaceutically acceptable salts, wherein R₁ is phenyl, naphthyl, benzoxazolonyl, indolyl, indolonyl, benzimidazolyl, quinolyl, furyl, benzofuryl, thienyl, benzothienyl, oxazolyl or benzoxazolyl;
R₂ is H or (C₁-C₆)alkyl;
R₃ is phenyl, pyridinyl, pyrimidinyl, pyrazinyl or pyridazinyl;
R₄ is H or (C₁-C₆)alkyl;
R₅ is H or (C₁-C₆)alkyl;
wherein each group of R₁ and R₃ may be independently and optionally substituted with one to four substituents independently selected from the groups consisting of fluoro, chloro, bromo, iodo, cyano, nitro, thiocyano, -SR₄, -SOR₄, -SO₂R₄, -NHSO₂R₄, -(C₁-C₆)alkoxy, -NR₄R₅, -NR₄COR₅, -CONR₄R₅, phenyl, -COR₄, -COOR₄, -(C₁-C₆)alkyl, -(C₁-C₆)alkyl substituted with one to six halogens, -(C₃-C₆)cycloalkyl and trifluoromethoxy;
X is O, S, SO, SO₂, NR₄, C=O, CH(OH), CHR₄, 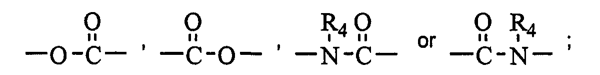
m is 0,1 or 2; and
n is 0, 1 or 2.

In certain embodiments, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula II, wherein R₁ is phenyl, naphthyl, benzoxazolonyl, indolyl, indolonyl, benzimidazolyl or quinolyl; wherein R₁ and R₃ may be independently substituted with up to three substituents independently selected from the group consisting of fluoro, chloro, bromo, iodo, cyano, -NR₄R₅, -(C₁-C₆)alkoxy, - COOR₄, -CONR₄R₅, -(C₁-C₆)alkyl, -(C₁-C₆)alkyl substituted with one to six halogens, -(C₃-C₆)cycloalkyl and trifluoromethoxy; R₂ is H or CH₃; X is O, C=O, CHOH, -C(=O)O- or CH₂; m is 0 or 1; n is 0 or 1; and the pharmaceutically acceptable salts thereof.

In other embodiments, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula II, wherein R₁ is phenyl or substituted phenyl; R₃ is substituted or unsubstituted phenyl, pyridinyl or pyrimidinyl; X is O, -C(=O)O- or CH2; and the pharmaceutically acceptable salts thereof; or R₂ is H; X is O; m is 0; n is 1; and the pharmaceutically acceptable salts thereof; or R₂ is H; X is O; m is 1; n is 0; and the pharmaceutically acceptable salts thereof; or R₂ is H; X is -C(=O)O-; m is 0; n is 0; and the pharmaceutically acceptable salts thereof; or R₁ is fluorophenyl, diflurophenyl or cyanophenyl; R₃ is chloropyridinyl; and the pharmaceutically acceptable salts thereof; or R₁ is fluorophenyl, diflurophenyl or cyanophenyl; R₃ is fluoropyrimidinyl; and the pharmaceutically acceptable salts thereof; or R₁ is fluorophenyl, diflurophenyl or cyanophenyl; R₃ is chloropyridinyl; and the pharmaceutically acceptable salts thereof; or R₁ is fluorophenyl, diflurophenyl or cyanophenyl; R₃ is fluoropyrimidinyl; and the pharmaceutically acceptable salts thereof; or R₁ is fluorophenyl, diflurophenyl or cyanophenyl; R₃ is chloropyridinyl; and the pharmaceutically acceptable salts thereof; or R₁ is fluorophenyl, diflurophenyl or cyanophenyl; R₃ is fluoropyrimidinyl; and the pharmaceutically acceptable salts thereof; or R₃ is 5-chloro-pyridin-2-yl-; and the pharmaceutically acceptable salts thereof; or R₃ is 5-fluoro-pyrimidin-2-yl-; and the pharmaceutically acceptable salts thereof; or R₃ is 5-chloro-pyridin-2-yl-; and the pharmaceutically acceptable salts thereof; or R₃ is 5-fluoro-pyrimidin-2-yl-; and the pharmaceutically acceptable salts thereof; or R₃ is 5-chloro-pyridin-2-yl-; and the pharmaceutically acceptable salts thereof; or R₃ is 5-fluoro-pyrimidin-2-yl-; and the pharmaceutically acceptable salts thereof.

Preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formula II and their pharmaceutically acceptable salts:
(7S,8aS)-7-(4-fluorophenoxy)methyl-2-(5-chloropyridin-2-yl)-1,2,3,4,6,7,8,8a-octahydro-pyrrolo[1,2-a]pyrazine;
(7S,8aS)-7-(3,5-d ifluorophenoxy)methyl-2-(5-chloropyridin-2-yl)-1,2,3,4,6,7,8,8a-octahydro-pyrrolo[1,2-a]pyrazine;
(7S,8aS)-7-(3-cyanophenoxy)methyl-2-(5-chloropyridin-2-yl)-1,2,3,4,6,7,8,8a-octahydro-pyrrolo[1,2-a]pyrazine;
(7S,8aS)-7-(4-cyanophenoxy)methyl-2-(5-chloropyridin-2-yl)-1,2,3,4,6,7,8,8a-octahydro-pyrrolo[1,2-a]pyrazine;
(7S,8aS)-7-(4-fluorobenzyl)oxy-2-(5-chloropyridin-2-yl)-1,2,3,4,6,7,8,8a-octahydro-pyrrolo[1,2-a]pyrazine;
(7S,8aS)-2-(5-chloropyridin-2-yl)-1,2,3,4,6,7,8,8a-octahydro-pyrrolo[1,2-a]pyrazin-7-yl benzoate;
(7S,8aS)-7-(4-fluorophenoxy)methyl-2-(5-fluoropyrimidin-2-yl)-1,2,3,4,6,7,8,8a-octahydro-pyrrolo[1,2-a]pyrazine;
(7S,8aS)-7-(3,5-difluorophenoxy)methyl-2-(5-fluoropyrimidin-2-yl)-1,2,3,4,6,7,8,8a-octahydro-pyrrolo[1,2-a]pyrazine;
(7S,8aS)-7-(3-cyanophenoxy)methyl-2-(5-fluoropyrimidin-2-yl)-1,2,3,4,6,7,8,8a-octahydro-pyrrolo[1,2-a]pyrazine;
(7S,8aS)-7-(4-cyanophenoxy)methyl-2-(5-fluoropyrimidin-2-yl)-1,2,3,4,6,7,8,8a-octahydro-pyrrolo[1,2-a]pyrazine;
(7S,8aS)-7-(4-fluorobenzyl)oxy-2-(5-fluoropyrimidin-2-yl)-1,2,3,4,6,7,8,8a-octahydro-pyrrolo[1,2-a]pyrazine;
(7S,8aS)-2-(5-fluoropyrimidin-2-yl)-1,2,3,4,6,7,8,8a-octahydro-pyrrolo[1,2-a]pyrazin-7-yl benzoate; and
(7S,8aS)-7-(3-cyanobenzyl)oxy-2-(5-fluoropyrimidin-2-yl)-1,2,3,4,6,7,8,8a-octahydro-pyrrolo[1,2-a]pyrazine.

In the compounds of formula II, unless otherwise indicated, the term "alkyl" includes saturated monovalent hydrocarbon radicals having straight, branched or cyclic moieties or combinations thereof.

Unless otherwise indicated, the term "alkoxy", as used in the compounds of formula II, refers to radicals having the formula -O-alkyl, wherein "alkyl" is defined as above for compounds of formula II.

Other examples of D4 receptor antagonists that may be used in the methods and pharmaceutical compositions of this invention are compounds of the formula 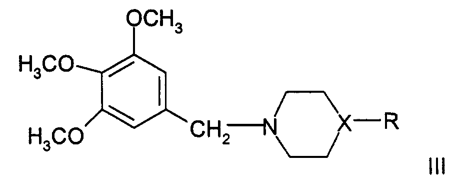 and their pharmaceutically acceptable salts, wherein X is N or CH; and
R is aryl or heteroaryl;
with the proviso that when X is N and R is aryl, aryl is not phenyl, phenyl monosubstituted by lower alkyl, lower alkoxy, halogen, or nitro, phenyl disubstituted by lower alkyl, or phenyl trisubstituted by lower alkoxy;
or compounds of the formula 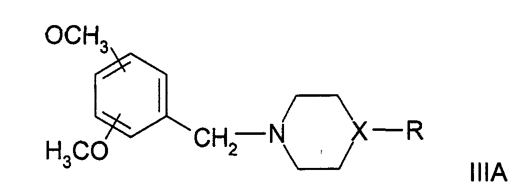 and their pharmaceutically acceptable salts, wherein X is N or CH; and
R is aryl or heteroaryl;
with the following provisos:
(a) that when X is N or CH, and R is aryl, aryl is not phenyl, or phenyl monosubstituted by lower alkyl, lower alkoxy, or halogen; and
(b) that when X is N and R is heteroaryl, heteroaryl is not 2-, 3-, or 4-pyridinyl.

In a preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula III, wherein R is phenyl, phenyl substituted by 1 to 3 substituents selected from the group consisting of lower alkyl, lower alkoxy, lower thioalkoxy, halogen, nitro, amino and cyano, 2-, 3-, or 4-pryridinyl, 4-, 5-, 6-, or 7-benzo[b]furanyl, 4-, 5-, 6-, or 7-benzo[b]thienyl, 4-, 5-, 6-, or 7-indolyl, 2-, 3-, 4-, 5-, 6-, 7-, or 8-quinolinyl, or 2-, 3-, 4-, 5-, 6-,7-, or 8-isoquinolinyl; with the proviso that when X is N, R is not phenyl, phenyl monosubstituted by lower alkyl, lower alkoxy, halogen or nitro, phenyl disubstituted by lower alkyl, or phenyl trisubstituted by lower alkoxy.

In a more preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula III, wherein R is phenyl, phenyl substituted by 1 to 2 substituents selected from the group consisting of lower alkyl, lower alkoxy and halogen, or 2-pyridinyl; with the proviso that when X is N, R is not phenyl, phenyl monosubstituted by lower alkyl, lower alkoxy or halogen, or phenyl disubstituted by lower alkyl.

In a most preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula III, wherein R is phenyl, phenyl substituted by 1 to 2 substituents selected from the group consisting of methyl, methoxy and chloro, or 2-pyridinyl; with the proviso that when X is N, R is not phenyl, phenyl monosubstituted by methyl, methoxy and chloro, or phenyl disubstituted by methyl.

Other preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formula III and their pharmaceutically acceptable salts:
1-(2, 5 -dichlorophenyl)-4-(3, 4, 5-trimethoxyhenzyl) piperazine;
1-(2, 3 -dichlorophenyl)-4-(3, 4, 5-trimethoxybenzyl) piperazine;
1 - (3, 4 -dichlorophenyl) - 4 - (3, 4, 5-trimethoxybenzyl) piperazine;
1-(2,3-dimethylphenyl)-4-(3,4,5-trimethoxybenzyl) piperazine;
1-(3, 4-dimethylphenyl)-4 - (3, 4, 5-trimethoxybenzyl) piperazine;
1 - (2-chloro-3-methylphenyl)-4-(3, 4, 5-trimethoxybenzyl) piperazine;
1-(2-chloro-4-methylphenyl)-4-(3, 4, 5-trimethoxybenzyl) piperazine;
1-(2-chloro-5-methylphenyl)-4-(3,4,5-trimethoxybenzyl) piperazine;
1-(3-chlor-2-methylphenyl)-4-(3, 4, 5-trimethoxybenzyl) piperazine;
1-(3-chloro-4-methylphenyl)-4-(3, 4, 5-trimethoxybenzyl) piperazine;
1-(5-chloro-2-methylphenyl)-4-(3, 4, 5-trimethoxybenzyl) piperazine
1-(4-chloro-2-methylphenyl)-4-(3, 4, 5-trimethoxybenzyl) piperazine;
1-(4-chloro-3-methylphenyl)-4-(3, 4, 5-trimethoxybenzyl) piperazine;
1-pyridin-2-yl-4-(3,4,5-trimethoxybenzyl) piperazine; and
4-phenyl-1-(3,4,5-trimethoxybenzyl)piperidine.

Additional preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formula III and their pharmaceutically acceptable salts:
1-phenyl-4- (3, 4, 5-trimethoxybenzyl) piperazine;
1-(2-chlorophenyl)-4-(3,4,5-trimethoxybenzyl) piperazine;
1-(3-chlorophenyl)-4-(3,4,5-trimethoxybenzyl) piperazine;
1-(4-chlorophenyl)-4-(3,4,5-trimethoxybenzyl) piperazine;
1-o-tolyl-4- (3, 4, 5-trimethoxybenzyl) piperazine;
1-m-tolyl-4-(3,4,5-trimethoxybenzyl)piperazine;
1-p-tolyl-4-(3,4,5-trimethoxybenzyl)piperazine;
1-(2-methoxyphenyl)-4-(3,4,5-trimethoxybenzyl) piperazine;
1-(3-methoxyphenyl)-4-(3,4,5-trimethoxybenzyl) piperazine;
1-(4-methoxyphenyl)-4-(3,4,5-trimethoxybenzyl) piperazine;
1-(2,5-dichlorophenyl)-4-(3,4,5-trimethoxybenzyl) piperazine;
1-(2,3-dichlorophenyl)-4-(3,4,5-trimethoxybenzyl) piperazine;
1-(3,4-dichlorophenyl)-4-(3,4,5-trimethoxybenzyl) piperazine;
1-(2,3-dimethylphenyl)-4-(3,4,5-trimethoxybenzyl) piperazine;
1-(3,4-dimethylphenyl)-4-(3,4,5-trimethoxybenzyl) piperazine;
1-(2-chloro-3-methylphenyl)-4-(3,4,5-trimethoxybenzyl) piperazine;
1- (2-chloro-4-methylphenyl) -4- (3, 4, 5-trimethoxybenzyl) piperazine;
1-(2-chloro-5-methylphenyl)-4-(3,4,5-trimethoxybenzyl) piperazine;
1- (3-chloro-2-methylphenyl) -4- (3, 4, 5-trimethoxybenzyl) piperazine;
1- (3-chloro-4-methylphenyl) -4- (3, 4, 5-trimethoxybenzyl) piperazine;
1-(5-chloro-2-methylphenyl)-4-(3,4,5-trimethoxybenzyl) piperazine;
1-(4-chloro-2-methylphenyl)-4-(3,4,5-trimethoxybenzyl) piperazine;
1-(4-chloro-3-methylphenyl)-4-(3,4,5-trimethoxybenzyl) piperazine;
1-pyridin-2-yl-4- (3,4,5-trimethoxybenzyl) piperazine; and
4-phenyl-1- (3,4,5-trimethoxybenzyl) piperidine.

In a preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IIIA, wherein R is phenyl, phenyl substituted by 1 to 3 substituents selected from the group consisting of lower alkyl, lower alkoxy, lower thioalkoxy, halogen, nitro, amino and cyano, 2-, 3-, or 4-pyridinyl, 4-, 5-, 6-, or 7-benzo[b]furanyl, 4-, 5-, 6-, or 7-benzo[b]thienyl, 4-, 5-, 6-, or 7-indolyl, 2-, 3-, 4-, 5-, 6-, 7-, or 8-quinolinyl, or 2-, 3-, 4-, 5-, 6-, 7-, or 8-isoquinolinyl; with the following provisos: (a) that when X is N or CH, R is not phenyl, or phenyl monosubstituted by lower alkyl, lower alkoxy or halogen, and (b) that when X is N, R is not 2-, 3-, or 4-pyridinyl.

In a more preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IIIA, wherein R is phenyl, phenyl substituted by 1 to 2 substituents selected from the group consisting of lower alkyl, lower alkoxy and halogen, or 2-pyridinyl; with the following provisos: (a) that when X is N or CH, R is not phenyl, phenyl monosubstituted by lower alkyl, lower alkoxy or halogen, and (b) that when X is N, R is not 2-pyridinyl.

In a most preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IIIA, wherein R is phenyl, phenyl substituted by 1 to 2 substituents selected from the group consisting of methyl, methoxy and chloro, or 2-pyridinyl; with the following provisos: (a) that when X is N or CH, R is not phenyl, phenyl monosubstituted by methyl, methoxy and chloro, and (b) that when X is N, R is not 2-pyridinyl.

Other preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formula IIIA and their pharmaceutically acceptable salts:
1- (2 -chloro- 3 -methyphenyl) - 4 - (2, 3 -dimethoxybenzyl) piperazine;
1-(2-chloro-3-methylphenyl)-4-(2,4-dimethoxybenzyl) piperazine;
1-(2-chloro-3-methylphenyl)-4-(2,5-dimethoxybenzyl) piperazine; and
1-(2-chloro-3 -methyphenyl)-4- (3, 4-dimethoxybenzyl) piperazine.

Additional preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formula IIIA and their pharmaceutically acceptable salts:
1- (2-chloro-3-methylphenyl) -4- (2, 3-dimethoxybenzyl) piperazine;
1- (2-chloro-3-methylphenyl) -4- (2, 4-dimethoxybenzyl) piperazine;
1-(2-chloro-3-methylphenyl)-4-(2,5-dimethoxybenzyl) piperazine; and
1-(2-chloro-3-methylphenyl) -4-(3,4-dimethoxybenzyl), piperazine.

In the compounds of formula III or formula IIIA, unless otherwise indicated, the term "lower alkyl" means a straight or branched hydrocarbon radical having from 1 to 6 carbon atoms and includes, for example, methyl, ethyl, n-propyl, isopropyl, n-butyl, sec-butyl, isobutyl, tert-butyl, n-pentyl, n-hexyl, and the like.

Unless otherwise indicated, the term "aryl", as used in the compounds of formula III or formula IIIA, means an aromatic radical which is a phenyl group or phenyl group substituted by 1 to 4 substituents selected from lower alkyl, lower alkoxy, lower thioalkoxy, halogen, nitro, amino, or cyano, such as for example, 2-chlorophenyl, 3-chlorophenyl, 4-chlorophenyl, 2-methylphenyl, 3-methylphenyl, 4-methylphenyl, 2-methoxyphenyl, 3-methoxyphenyl, 4-methoxyphenyl, 2-chloro-3-methylphenyl, 2-chloro-4-methylphenyl, 2-chloro-5-methylphenyl, 3-chloro-2-methylphenyl, 3-chloro-4-methylphenyl, 4-chloro-2-methylphenyl, 4-chloro-3-methylphenyl, 5-chloro-2-methylphenyl, 2,3-dichlorophenyl, 2,5-dichlorophenyl, 3,4-dichlorophenyl, 2,3-dimethylphenyl, 3,4-dimethylphenyl, and the like.

Unless otherwise indicated, the term "heteroaryl", as used in the compounds of formula III or formula IIIA, means a heteroaromatic radical which is 2-, 3- or 4-pyridinyl, 4-,5-, 6-, or 7-benzo[b]furanyl, 4-, 5-,6-, or 7-benzo[b]thienyl, 4-,5-, 6-, or 7-indolyl, 2-, 3-, 4-,5-, 6-, 7-, or 8-quinolinyl, 2-, 3-,4-, 5-, 6-, 7-, or 8-isoquinolinyl.

Unless otherwise indicated, the terms "lower alkoxy" and "lower thioalkoxy", as used in the compounds of formula III or formula IIIA, are, respectively, O-alkyl or S-alkyl of from 1 to 6 carbon atoms as defined above for "lower alkyl" for compounds of formula III or formula IIIA.

Unless otherwise indicated, the term "halogen", as used in the compounds of formula III or formula IIIA, includes fluorine, chlorine, bromine and iodine.

Other examples of D4 receptor antagonists that may be used in the methods and pharmaceutical compositions of this invention are compounds of the formula 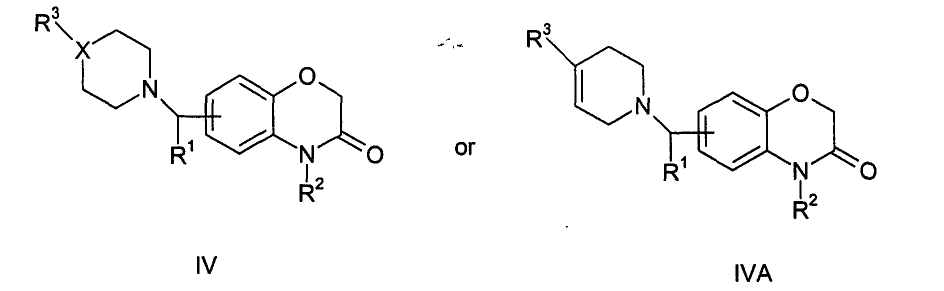 and their pharmaceutically acceptable salts, esters, amides and prodrugs thereof, wherein R¹ and R² are independently hydrogen or C₁-C₆ alkyl;
X is N or CH; and
R³ is phenyl, naphthyl, heteraryl, substituted phenyl, substituted naphthyl or substituted heteroaryl, wherein each substituent is independently selected from halogen, C₁-C6 alkoxy, C₁-C₆ alkyl, -CN, -CF₃ or sulphonamido.

The atoms in the benzoxazinone group can be numbered as shown below: 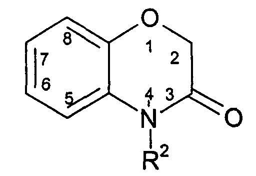 In a preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IV or IVA, wherein the group 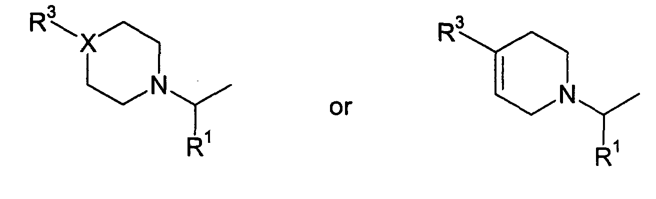 is attached to the benzoxazinone group at the 6 or 7 position.

In another preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IV or IVA, wherein R¹ and R² are hydrogen.

In another preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IV or IVA, wherein R³ is phenyl, methyltolyl, tolyl, or sulfonamido.

In another preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IV or IVA, wherein X is N. N.

Other examples of D4 receptor antagonists that may be used in the methods and pharmaceutical compositions of this invention are compounds of the formula 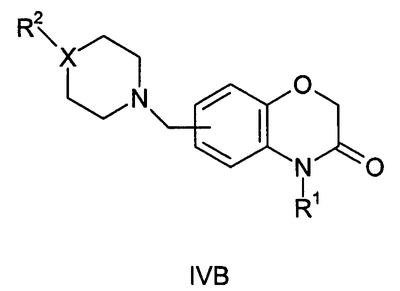 and their pharmaceutically acceptable salts, esters, amides and prodrugs thereof, wherein X is N or CH;
R¹ is hydrogen or methyl; and
R² is phenyl or substituted phenyl, wherein each substituent is independently selected from C₁-C₆ alkyl or sulphonamido.

In a preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IVB, wherein the group, 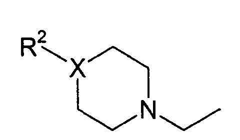 is attached to the benzoxazinone group at the 6 or 7 position.

In another preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IVB, wherein R¹ is hydrogen.

In another preferred embodiment, this invention relates to the above pharmaceutical compositions for the treatment of depression, anxiety or psychosis, and the above methods of treating depression, anxiety or psychosis, wherein the D4 receptor antagonist, or pharmaceutically acceptable salt thereof, is a compound of the formula IVB, wherein R² is phenyl, methyltolyl, tolyl, or sulfonamido.

More preferred embodiments of this invention relate to the above pharmaceutical compositions for the treatment of depression, anxiety or psychosis, and the above methods of treating depression, anxiety or psychosis, wherein the D4 receptor antagonist, or pharmaceutically acceptable salt thereof, is selected from the following compounds of the formulas IV, IVA and IVB and their pharmaceutically acceptable salts:
4-(4-(3-oxo-3,4-dihydro-2H-benzo[1,4]oxazin-6-ylmethyl)-piperazin-1-yl]-benzenesulfonamide;
6-[4-(3, 4-dimethyl-phenyl)-piperazin-1-ylmethyl]-4H-benzo[1,4]oxazin-3-one;
6-(4-p-tolyl-piperazin-1-ylmethyl)-4H-benzo[1,4]oxazin-3-one;
6-[4-phenyl-piperazin-1-ylmethyl]-4H-benzo[1,4]oxazin-3-one;
7-(4-p-tolyl-piperazin-I-ylmethyl -4H-benzo[1,41 oxazin-3-one;
7-(4-phenyl-piperazin-I-ylmethyl)-4H-benzo[1,4]oxazine-3-one;
7-[4-(3,4-dimethyl-phenyl)-piperazin-1-ylmethyl)-4H-benzo[1,4]oxazine-3-one;
6-[4-(5-methyl-pyridin-2-yl)-piperazin-1-ylmethyl]-4H-benzo[1,4]oxazin-3-one;
6-(4-p-tolyl-piperidin-1-ylmethyl)-4H-benxo[1,4]oxazin-3-one;
6-[4-(3,4-Dimethyl-phenyl)piperidin-1-ylmethyl]-4H-benzo[1,4]oxazin-3-one;
6-(4-thiazol-2-yl-piperazin-1-ylmethyl)-4H-benzo[1,4]oxazin-3-one;
6-(4-benzothiazol-2-yl-piperazin-1-ylmethyl)-4H-benzo[1,4]oxazin-3-one;
6-(4-(4,5-dimethyl-thiazol-2-yl)-piperazin-1-ylmethyl]-4H-benzo[1,4]oxazin-3-one;
6-(4-naphthalen-2-yl-piperazin-1-ylmethyl)-4H-benzo[1,4]oxazin-3-one;
6-[4-(3-chloro-phenyl)-piperazin-1-ylmethyl]-4H- benzo[1,4]oxazin-3-one;
6-[4-(3,4-dichloro-phenyl)-piperaziin-1-ylmethyl)-4H-benzo[1,4]oxazin-3-one;
2-[4-(3-oxo-3,4-d ihydro-2H-benzo[1,4]oxazin-6-ylmethyl)-piperazin-1-yl]-benzonitrile;
6-[4-(4-methoxy-phenyl)-piperazin-1-ylmethyl]-4H-benzo[1,4]oxazin-3-one;
6-[4-(2-chloro-4-methyl-phenyl)-piperazin-1-ylmethyl]-4H-benzo[1,4]oxazin-3-one;
6-(4-(4-Fluoro)-phenyl)-piperazin-1-ylmethyl]-4H-benzo[1,4]oxazin-3-one;
6-[4-(3-Trifluoromethyl-phenyl)-piperazin-1-ylmethyl]-4H-benzo[1,4]oxazin-3-one;
6-[4-(3,5-Dimethyl-phenyl)-piperazin-1-ylmethyl]-4H-benzo[1,4]oxazin-3-one;
6-[4-(2-Chloro-phenyl)-piperazin-1-ylmethyl]-4H-benzo[1,4]oxazin-3-one;
6-[4-(4-Trifluoromethyl-phenyl)-piperazin-1-ylmethyl]-4H-benzo[1,4]oxazin-3-one;
6-[4-(4-Chloro-phenyl)-piperazin-1-ylmethyl]-4H-benzo[1,4]oxazin-3-one;
7-[4-(5-Methyl-pyridin-2-yl)-piperazin-1-ylmethyl]-4H-benzo[1,4]oxazin-3-one;
7-[4-(4-Methoxy-phenyl)-piperazin-1-ylmethyl]-4H-benzo[1,4]oxazin-3-one,
7-[4-(4-Chloro-phenyl)-piperazin-1-ylmethyl]-4H-benzo[1,4]oxazin-3-one;
7-[4-(3,4-Dimethyl-phenyl)-piperidin-1-ylmethy]-4H-benzo[1,4]oxazin-3-one;
6-[4-(4-Methoxy-phenyl)-piperidin-1-ylmethyl]-4H-benzo[1,4]oxazin-3-one;
7-[4-(4-Methoxy-phenyl)-piperidin-1-ylmethyl]-4H-benzo[1,4]oxazin-3-one;
7-(4-Phenyl-piperidin-1-ylmethyl)-4H-benzo[1,4]oxazin-3-one;
7-(4-Naphthalen-2-yl-piperazin-1-ylmethyl)-4H-benzo[1,4]oxazin-3-one; and
7-(4-p-Tolyl-piperidin-1-ylmethyl)-4H-benzo[1,4]oxazin-3-one.

Unless otherwise indicated, the term "alkyl", as used in the compounds of formula IV, IVA and IVB, means a straight or branched chain hydrocarbon. Representative examples of alkyl groups are methyl, ethyl, propyl, isopropyl, isobutyl, butyl, tert-butyl, sec-butyl, pentyl and hexyl.

Unless otherwise indicated, the term "aryl", as used in the compounds of formula IV, IVA and IVB, means a cyclic aromatic hydrocarbon. Representative examples of aryl groups include phenyl and naphthyl, which can be substituted or unsubstituted. Examples of suitable substituents include halogen, C₁-C₆ alkyl, hydroxy, C₁-C₆ alkoxy, -CF₃ and sulfonamides.

Unless otherwise indicated, the term "heteroaryl", as used in the compounds of formula IV, IVA and IVB, means a cyclic hydrocarbon that contains one or more heteroatoms. Representative examples of heteroaryl groups are thiazole, thiophene, pyridine, pyrimidine, quinoline, isoquinoline and imidazole. The heteroaryl group can be substituted or unsubstituted. Examples of suitable substituents include C₁-C₆ alkyl, C₁-C₆ alkoxy or halogen.

Unless otherwise indicated, the term "heteroatom", as used in the compounds of formula IV, IVA and IVB, means an atom other than carbon. Examples of heteroatoms include nitrogen, oxygen, sulfur and phosphorus.

Unless otherwise indicated, the term "halogen", as used in the compounds of formula IV, IVA and IVB, means chlorine, fluorine, bromine and iodine.

Unless otherwise indicated, the term "sulfonamido", as used in the compounds of formula IV, IVA and IVB, means a group having the structure -SO₂NR^{a}R^{b}, where R^{a} and R^{b} are sulfonamido substituents well known to those in the art such as hydrogen and C₁-C₆ alkyl.

Unless otherwise indicated, the symbol "-", as used in the compounds of formula IV, IVA and IVB, means a bond.

The term "pharmaceutically acceptable salts, esters, amides, and prodrugs", as used with regard to the compounds of formulas IV, IVA and IVB, refers to those carboxylate salts, amino acid addition salts, esters, amides, and prodrugs of the compounds of formulas IV, IVA and IVB which are, within the scope of sound medical judgement, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention.

Examples of pharmaceutically acceptable, nontoxic esters of the compounds of formulas IV, IVA and IVB include C₁-C₆ alkyl esters wherein the alkyl group is a straight or branched chain. Acceptable esters also include C₅-C₇ cycloalkyl esters as well as arylalkyl esters such as, but not limited to, benzyl. C₁-C₄ alkyl esters are preferred. Esters of the compounds of the present invention may be prepared according to conventional methods.

Examples of pharmaceutically acceptable, nontoxic amides of the compounds of formulas IV, IVA and IVB include amides derived from ammonia, primary C₁-C₆ alkyl amines and secondary C₁-C₆ dialkyl amines wherein the alkyl groups are straight or branched chains. In the case of secondary amines the amine may also be in the form of 5- or 6-membered heterocycle containing one nitrogen atom. Amides derived from ammonia, C₁-C₃ alkyl primary amines and C₁-C₂ dialkyl secondary amines are preferred. Amides of the compounds of the invention may be prepared according to conventional methods.

The term "prodrug" refers to compounds that are rapidly transformed *in vivo* to yield the parent compound of the formulas IV, IVA and IVB, for example, by hydrolysis in blood. A thorough discussion is provided in T. Higuchi and V. Stella, "Pro-drugs as Novel Delivery Systems," Vol 14 of the A.C.S. Symposium Series, and in Bioreversible Carriers in Drug Design, ed. Edward B. Roche, American Pharmaceutical Association and Pergamon Press, 1987, both of which are incorporated herein by reference.

Another example of a D4 receptor antagonist that may be used in the methods and pharmaceutical compositions of this invention is L-745,870 (3-(4-(4-chlorophenyl)piperazin-1-yl)methyl-1H-pyrrolo(2,3-b)pyridine), the activity and synthesis of which is referred to in U.S. Patent No. 5,432,177, issued July 11, 1995, International Patent Application No. WO 94/20497, published September 15, 1994 and U.S. Patent No. 5,622,950, issued April 22, 1997, and U.S. Patent No. 5,563,152, issued October 8, 1996, International Patent Application No. WO 94/20459, published September 15, 1994, U.S. Patent No. 5,563,150, issued October 8, 1996, International Patent Application No. WO 96/05200, published February 22, 1996, and Kulagowski, J.J., Broughton, H.B., Curtis, N.R., Mawer, I.M., Ridgill, M.P., Baker, R., *et al.,* "3-[[4-(4-Chlorophenyl)piperazine-1-yl]methyl]-1H-pyrrolo[2,3-b]pyridine: an antagonist with high affinity and selectivity for the human dopamine receptor," J. Med. Chem., 39, 1941-1942, (1996), all of which are incorporated herein by reference in their entirety.

Another example of a D4 receptor antagonist that may be used in the methods and pharmaceutical compositions of this invention is NGD 941, 2-(4-(2-phenylimidazol-5-ylmethyl)piperazin-1-yl)pyrimidine, 2-(4-((2-phenyl-1H-imidazol-4-yl)methyl)-1piperazinyl)pyrimidine, the activity and synthesis of which is referred to in U.S. Patent No. 5,633,376, issued May 27, 1997, U.S. Patent No. 5,428,164, issued June 27, 1995 and International Patent Application No. WO 96/10018, published April 4, 1996, and International Patent Application No. WO 96/160,040, published May 30, 1996, and Thurkauf, A., Yuan, J., Chen, X., He, X.S., Wasley, J.W.F., Hutchinson, A., et *al*., "2-Phenyl-4(5)-[[4-(pyrimidin-2-yl)piperazine-1-yl]methyl]imidazole: a highly selective antagonist at cloned human D4 receptors," J. Med. Chem., 40, 1-3, 1997, Thurkauf, A., "The synthesis of tritiated 2-phenyl-4-[4-(2-pyrimidyl)piperazinyl]methylimidazole ([³H] NGD 94-1): a ligand selective for the dopamine D4 receptor subtype," J. Lab. Comp. Radiopharm., 39, 123-128, 1997 and Primus, R.J., Thurkaur, A., Xu, J., Yevich, E., Mclnerney, S., Shaw, K., et *al*., "Localization and characterization of dopamine D4 binding sites in rat and human brain by use of the novel D4 receptor-selective ligand [³H]NGD 94-1," J. Pharmacol. Exp. Ther., 282, 1020-1027, 1997, all of which are incorporated herein by reference in their entirety.

Another example of a D4 receptor antagonist that may be used in the methods and pharmaceutical compositions of this invention is the active metabolite of NGD 941, the activity and synthesis of which is referred to in U.S. Patent No. 5,681,956, issued October 28, 1997, and "Neurogen update on schizophrenia program," Drug News Persp., 8, 559, 1995.

Another example of a D4 receptor antagonist that may be used in the methods and pharmaceutical compositions of this invention is PNU-101,387 (sonepiprazole), (S)-4-(4-(2-(3,4-dihydro-1H-2-benzopyran-1-yl)ethyl)-1-piperazinyl benzenesulfonamide, (S)-4(4-(2-(isochroman-1-yl)ethyl)piperazin-1-yl)benzenesulfonamide, the activity and synthesis of which is referred to in U.S. Patent No. 5,877,317, issued March 2, 1999 and International Patent Application WO 95/18118, published July 6, 1995, and Schlachter, S.K., Poel, T.J., Lawson, C.F., Dinh, D.M., Lajiness, M.E., Romero, A.G., *et al.,* "Substituted 4-aminopiperidines having high *in vitro* affinity and selectivity for the cloned human dopamine D4 receptor," Eur. J. Pharmacol., 322, 283-286, 1997, Kula, N.S., Baldessaarini, R.J., Kebabian, J.W., Bakthavachalam, V., Xu, L., "RBI-257: a highly potent dopamine D4 receptor-selective ligand," Eur. J. Pharmacol., 331, 333-336, 1997, Ten Brink, R.E., Bergh, C.L., Duncan, J.N., Harris, D.W., Huff, R.M., Lahti, R.A., et *al.,* "(S)-(-)-4-[4-[2-(lsochroman-1-yl)ethyl]piperazin-1-yl]benzenesulfonamide: a selective dopamine D4 antagonist," J. Med. Chem., 39, 2435-2437, 1996, and Merchant, K.M., Gill, G.S., Harris, D.W., Huff, R.M., Eaton, M.J., Lookingland, K., *et al.,* "Pharmacological characterization of U-101387: a dopamine D4 receptor selective antagonist," J. Pharmacol. Exp. Ther., 279, 1392-1403, 1996, all of which are incorporated herein by reference in their entirety.

Another example of a D4 receptor antagonist that may be used in the methods and pharmaceutical compositions of this invention is LU-111,995 (balaperidone, (1α,5α,6α)-3-(2-(6-(4-fluorophenyl)-3-azabicyclo(3.2.0)hept-3-yl0ethyl)-2,4(1H,3H)-quinazolinedione), the activity and synthesis of which is referred to in U.S. Patent No. 5,475,105, issued October 10, 1995 and International Patent Application No. WO 94/00458, published January 6, 1994, and Steiner, G., Bach, A., Bialojan, S., Greger, G., Hoger, T., Klebe, G., et al., "LU111995: a novel D4/5HT2 receptor antagonist and potential new antipsychotic," American Chemical Society 213^{th} National Meeting, San Francisco, California, USA, MEDI 186, 1997, all of which are incorporated herein by reference in their entirety.

Another example of a D4 receptor antagonist that may be used in the methods and pharmaceutical compositions of this invention is RP 62203 (fananserin, 2-(3-(4-(4-fluorophenyl)-1-piperazinyl)propyl)naphtho(1,8-c,d)isothiazole-1,1-dioxide), the activity and synthesis of which is referred to in U.S. Patent No. 5,021,420, issued June 4, 1991, which is incorporated herein by reference in its entirety.

Another example of a D4 receptor antagonist that may be used in the methods and pharmaceutical compositions of this invention is PNU-96,415E, the activity and synthesis of which is referred to in Tang, A.H., Franklin, S.R., Himes, C.S., Smith, M.W., Ten Brink, R.E., "PNU-96415E: a potential antipsychotic agent with clozapine-like pharmacological properties," J. Pharmacol. Exp. Ther., 281, 440-447, 1997, which is incorporated herein by reference in its entirety.

Another example of a D4 receptor antagonist that may be used in the methods and pharmaceutical compositions of this invention is olanzapine, the activity and synthesis of which is referred to in U.S. Patent No. 5,229,382, issued July 20, 1993, and U.S. Patent No. 4,115,574, issued September 19, 1978, and U.S. Patent No. 5,736,541, issued April 7, 1998, U.S. Patent No. 5,919,485, issued July 6, 1999, U.S. Patent No. 5,817,656, issued June 10, 1998, U.S. Patent No. 5,817,655, issued June 10, 1998, U.S. Patent No. 5,627,178, issued May 6, 1997, U.S. Patent No. 5,605,897, issued February 25, 1997, and Bymaster, F.P., Calligaro, D.O., Falcone, J.F., Marsh, R.D., Moore, N.A., Tye, N.C., et *al.,* "Radioreceptor binding profile of the atypical antipsychotic olanzapine," Neuropsychopharmacology, 14, 87-96, 1996, all of which are incorporated herein by reference in their entirety.

Other examples of D4 receptor antagonists that may be used in the methods and pharmaceutical compositions of this invention are the compounds referred to in the following references, which references refer to methods of preparing same, and their pharmaceutically acceptable salts: U.S. Patent No. 5,883,094, issued March 16, 1999 and International Patent Application No. WO 95/34555, published December 21, 1995; U.S. Patent No. 5,889, 010, issued March 30, 1999 and International Patent Application No. WO 96/04250, published February 15, 1996; International Patent Application No. WO 98/08835, published March 5, 1998; European Patent Application EP0953567; International Patent Application No. WO 99/09025, published February 25, 1999; GB 2306164; International Patent Application Nos. WO 94/21630, published September 29, 1994 and WO 94/21626, published September 29, 1994; International Patent Application Nos. WO 94/21627, published September 29, 1994 and WO 94/21628, published September 29, 1994; International Patent Application No. WO 95/29911, published November 9, 1995; International Patent Application Nos. WO 94/22839, published October 13, 1994 and WO 94/21615, published September 29, 1994; International Patent Application No. WO 94/24105, published October 27, 1994; International Patent Application No. WO 95/14690, published June 1, 1995 and U.S. Patent No. 5,614,518, issued March 25, 1997; International Patent Application No. WO 94/20471, published September 15, 1994; International Patent Application No. WO 95/07904, published March 23, 1995; Rowley, M., Broughton, H.B., Collins, I., Baker, R., Emms, F., Marwood, R., *et al*., "5-(4-Chlorophenyl)-4-methyl-3-(1-(2-phenylethyl)piperidine-4-yl)isoxazole: a potent, selective antagonist at human cloned dopamine receptors," J. Med. Chem., 39, 1943-1945, 1996, Rowley, M., Collins, I., Broughton, H.B., Davey, W.B., Baker, R., Emms, F., *et al.,* "4-Heterocyclylpiperidines as selective high-affinity ligands at the human dopamine D4 receptor," J. Med. Chem., 40, 2374-2385, 1997, and International Patent Application No. WO 94/10145, published May 11, 1994; International Patent Application No. WO 96/21660, published July 18, 1996; International Patent Application No. WO 94/10162, published May 11, 1994; International Patent Application No. WO 95/07893, published March 23, 1995; International Patent Application No. WO 95/07262, published March 16, 1995; International Patent Application No. WO 95/14672, published June 1, 1995; Rowley, M., Collins, I., Broughton, H.B., Davey, W.B., Baker, R., Emms, F., *et al.,* "4-Heterocyclylpiperidines as selective high-affinity ligands at the human dopamine D4 receptor," J. Med. Chem., 40, 2374-2385, 1997, and International Patent Application No. WO 94/26733, published November 24, 1994; GB2310376; GB2311010; Kulagowski, J.J., Patel, S., "Dopamine D4 receptor antagonists," Curr. Pharm. Design, 3, 355-366, 1997; U.S. Patent No. 5,700,941, issued December 23, 1997; U.S. Patent No. 5,478,934, issued December 26, 1995; International Patent Application No. WO 96/16057, published May 30, 1996; International Patent Application No. WO 96/25411, published August 22, 1996; International Patent Application No. 96/16058, published May 30, 1996; U.S. Patent Nos. 5,656,632, issued August 12, 1997 and 5,602,168, issued February 11, 1997 and International Patent Application No. WO 96/39403, published December 12, 1996; International Patent Application No. WO 96/25414, published August 22, 1996; International Patent Application No. WO 96/41630, published December 27, 1996; International Patent Application No. WO 96/41629, published December 27, 1996; International Patent Application No. WO 96/35666, published November 14, 1996; International Patent Application No. WO 97/13759, published April 17, 1997; International Patent Application Nos. WO 96/18621, published June 20, 1996 and WO 96/18623, published June 20, 1996; International Patent Application Nos. WO 96/18630, published June 20, 1996, WO 95/17400, published June 29, 1995 and WO 96/18622, published June 20, 1996; Okuyama, S., Chaki, S., Kawashima, N., Suzuki, Y., Ogawa, S.-I., Kuagai, T., *et al.,* "The atypical antipsychotic profile of NRA0045, a novel dopamine D4 and 5-hydroxytryptamine_{2A} receptor antagonist, in rats," Br. J. Pharmacol., 121, 515-525, 1997, Okuyama, S., Chaki, S., Yoshikawa, R., Suzuki, Y., Ogawa, S.-I., Imagawa, Y., *et al.,* "*In vitro* and *in vivo* characterization of the dopamine D4 receptor, serotonin 5-HT2A receptor and *alpha-1* adrenoceptor antagonist (R)-(+)-2-amino-4-(4-fluorophenyl)-5-[1-[4-(4-fluorophenyl)-4-oxobutyl]pyrrolidin-3-yl]thiazole (NR0045)," J. Pharmacol. Exp. Ther., 282, 56-63, 1997, and International Patent Application No. WO 96/29330, published September 26, 1996; Ohmori, J., Maeno, K., Kidaka, K., Nakato, K., Matsumoto, M., Taka, S., *et al*., "Dopamine D3 and D4 receptor antagonists: synthesis and structure-activity relationships of (S)-(+)-N-(1-benzyl-3-pyrrolidinyl)-5-chloro-4-[(cyclopropylcarbonyl)amino]-2-methoxybenzamide (YM-436110 and related compounds," J. Med. Chem., 39, 2764-2772, 1996, and International Patent Application No. WO 95/08533, published March 30, 1995; Hidaka, K., Tada, S., Matsumoto, M., Ohmori, J., Maeno, K., Yamaguchi, T., *et al*., "YM-50001: a novel, potent and selective dopamine D4 receptor antagonist," NeuroReport, 7, 2543-2546, 1996; International Patent Application No. WO 97/03986, published February 6, 1997; Boyfield, I., coldwell, M.C., Hadley, M.C., Healy, M.A.M., Johns, A., Nash, D.J., *et al*., N-(Substituted-phenyl)piperazines: antagonists with high binding and functional selectivity for dopamine D4 receptors," Bioorg. Med. Chem. Lett., 6, 1227-1232, 1996, and Boyfield, I. Brown, T.H. Coldwell, M.C., Cooper, D.G., Hadley, M.S., Hagan, J.J., *et al.*, "Design and synthesis of 2-naphthoate esters as selective dopamine D4 antagonists," J. Med. Chem., 39, 1946-1948, 1996; and International Patent Application Nos. WO 97/43279, published November 20, 1997 and WO 97/43271, published November 20, 1997. The foregoing patents, patent applications and articles are incorporated herein by reference in their entirety.

The terms "anxiolytic effective amount" and "antianxiety effective amount", as used herein, refer to an amount that is effective in treating anxiety.

The term "antidepressant effective amount", as used herein, refers to an amount that is effective in treating depression.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The pharmaceutical compositions and methods of this invention comprise, or comprise administering, D4 receptor antagonists of the formulas I through IVB, which may have chiral centers and therefore exist in different enantiomeric forms. This invention includes methods and pharmaceutical compositions, as described above, wherein the D4 receptor antagonists that are employed are optical isomers, tautomers or stereoisomers of the compounds of formulas I through IVB that are defined above, or mixtures thereof.

Those D4 receptor antagonists of the formulas I through IVB that contain basic groups can form acid addition salts with various inorganic and organic acids. The present invention also relates to pharmaceutical compositions and methods comprising, or comprising administering, pharmaceutically acceptable acid addition salts of D4 receptor antagonists and of antidepressant and anxiolytic agents. The possible acids which are used to prepare the pharmaceutically acceptable acid addition salts of the basic active agents employed in the methods and pharmacuetical compositions of this invention are those which form non-toxic acid addition salts, *i.e.,* salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i.e.*, 1,1'-methylene-bis-(2-hydroxy-3- naphthoate)]salts.

Those D4 receptor antagonists of the formulas I through IVB that contain acidic groups can form base addition salts with certain bases. The present invention also relates to pharmaceutical compositions and methods comprising, or comprising administering, pharmaceutically acceptable base addition salts of D4 receptor antagonists and of antidepressant and anxiolytic agents. The chemical bases that may be used as reagents to prepare pharmaceutically acceptable base salts of the acidic active agents that are employed in the methods of this invention are those that form non-toxic base salts with such compounds. Such non-toxic base salts include, but are not limited to, those derived from such pharmacologically acceptable cations such as alkali metal cations (*e.g.*, potassium and sodium) and alkaline earth metal cations (*e.g.*, calcium and magnesium), ammonium or water-soluble amine addition salts such as N-methylglucamine (meglumine), and the lower alkanolammonium and other base salts of pharmaceutically acceptable organic amines.

The present invention also relates to pharmaceutical compositions and methods of treatment that employ isotopically-labeled compounds that are identical to those recited in formulas I through IVB, or to other D4 receptor antagonists, but for the fact that one or more atoms are replaced by an atom having an atomic mass or mass number different from the atomic mass or mass number usually found in nature. Examples of isotopes that can be incorporated into the D4 receptor antagonists that are employed in the pharmaceutical compositions and methods of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The D4 receptor antagonists employed in the pharmaceutical compositions and methods of the present invention, prodrugs thereof, and pharmaceutically acceptable salts of said compounds or of said prodrugs which contain the aforementioned isotopes and/or other isotopes are within the scope of this invention. Certain isotopically-labeled D4 receptor antagonists, for example, those into which radioactive isotopes such as ³H and ¹⁴C are incorporated, are useful in drug and/or substrate tissue distribution assays. Tritiated, *i.e.,* ³H, and carbon-14, *i.e.*, ¹⁴C, isotopes are particularly preferred for their ease of preparation and detectability. Further, substitution with heavier isotopes such as deuterium, *i.e.,* ²H, can afford certain therapeutic advantages resulting from greater metabolic stability, for example increased *in vivo* half-life or reduced dosage requirements and, hence, may be preferred in some circumstances.

### Detailed Description Of The Invention

D4 receptor antagonists of the formula I can be prepared as described in International Patent Application No. WO 96/10571, published April 11, 1996, and as described in U.S. Patent No. 5,852,031, issued December 22, 1998, and also as described in European Patent Application EP 0783503. The foregoing patents and patent applications are incorporated herein by reference in their entirety.

D4 receptor antagonists of the formula II can be prepared as described in International Patent Application No. WO 97/23482, published November 6, 1996, and as described in U.S. Patent No. 5,714,487, issued February 3, 1998. The foregoing patents and patent applications are incorporated herein by reference in their entirety.

D4 receptor antagonists of the formula III and IIIA can be prepared as described in International Patent Application No. WO 97/41108, published November 6, 1997. The foregoing patent application is incorporated herein by reference in its entirety.

D4 receptor antagonists of the formula IV, IVA and IVB can be prepared as described in International Patent Application No. WO 97/45419, published December 4, 1997. The foregoing patent application is incorporated herein by reference in its entirety.

This invention relates both to methods of treating depression, anxiety or psychosis, in which the D4 receptor antagonist and the antidepressant or anxiolytic agent, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the D4 receptor antagonist will be administered to an adult human in an amount ranging from 0.05 to 1500 mg per day, in single or divided doses, preferably from 5 to 500 mg/day. The compounds may be administered on a regimen of up to 6 times per day, preferably 1 to 4 times per day, especially 2 times per day and most especially once daily. A suitable dosage level for the antidepressant agent is 0.5 to 1500 mg per day, preferably 2.5 to 1000 mg per day, and especially 2.5 to 750 mg per day. The compounds may be administered on a regimen of up to 6 times per day, preferably 1 to 4 times per day, especially 2 times per day and most especially once daily. A suitable dosage level for the anxiolytic agent is 0.1 to 1500 mg per day, preferably 0.1 to 1000 mg per day, and especially 0.1 to 500 mg per day. The compounds may be administered on a regimen of up to 6 times per day, preferably 1 to 4 times per day, especially 2 times per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day. Compounds that have been approved for use by the Food and Drug Administration (FDA) will have preferred dosages as indicated in the most current edition of the *Physicians' Desk Reference* (PDR® , published by Medical Economics Company, Inc), which, as of the filing date of this application, is the 54^{th} Edition.

The D4 receptor antagonists, their pharmaceutically acceptable salts, and the antidepressant and anxiolytic agents and their pharmaceutically acceptable salts that are employed in the pharmaceutical compositions and methods of this invention are hereinafter also referred to as "therapeutic agents". The therapeutic agents can be administered via either the oral or parenteral route. Compositions containing both a D4 receptor antagonist and an antidepressant or anxiolytic agent, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i.e.,* they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, *etc.* Moreover, oral pharmaceutical compositions can be suitably sweetened and/or flavored. In general, the therapeutic agents of this invention, when administered separately *(i.e.,* not in the same pharmaceutical composition) are present in such dosage forms at concentration levels ranging from 5.0% to 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the D4 receptor antagonist and the antidepressant or anxiolytic agent may be formulated in a single pharmaceutical composition or alternatively in individual pharmaceutical compositions for simultaneous, separate or sequential use in accordance with the present invention.

Preferably the compositions according to the present invention, which contain both a D4 receptor antagonist and an antidepressant or an anxiolytic agent, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.,* conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from 0.05 to 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavoured syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of a D4 receptor antagonist or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g*., Tween™ 20, 40, 60, 80 or 85) and other sorbitans (*e.g*., Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™, Lipofundin ™ and Lipiphysan™. The therapeutic agent may be either dissolved in a pre-mixed emulsion composition or alternatively it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising a D4 receptor antagonist and an antidepressant or anxiolytic agent, or pharmaceutically acceptable salts of the same, which process comprises bringing a D4 receptor antagonist and the antidepressant or anxiolytic agent (or the pharmaceutically acceptable salts of one or both of these therapeutic agents) into association with a pharmaceutically acceptable carrier or excipient.

It will be appreciated that the amount of the D4 receptor antagonist and the antidepressant or anxiolytic agent required for use in the treatment of depression, anxiety or psychosis will vary not only with the particular compounds or compositions selected but also with the route of administration, the nature of the condition being treated, and the age and condition of the patient, and will ultimately be at the discretion of the patient's physician or pharmacist.

Dopaminergic activity of the compounds used in the invention is related to the ability of the compounds to bind to the D4 receptors, and the relative ability of compounds of this invention to inhibit [³H]-spiperone binding to human dopamine D4 receptor subtypes expressed in clonal cell lines was measured using the following procedure.

The determination of D4 receptor binding ability has been described by Van Tol, *et al.,* Nature, 350, 610 (1991)). Clonal cell lines expressing the human dopamine D4 receptor are harvested and homogenized (polytron) in a 50 mM Tris:HCI (pH 7.4 at 4°C) buffer containing 5 mM EDTA, 1.5 mM calcium chloride (CaCl₂), 5 mM magnesium chloride (MgCl₂), 5mM potassium chloride (KCI) and 120 mM sodium chloride (NaCI). The homogenates are centrifugated for 10-15 min. at 48,000 g, and the resulting pellets resuspended in a buffer at a concentration of 150-250 mg/ml. For saturation experiments, 0.75 ml aliquots of tissue homogenate are incubated in triplicate with increasing concentrations of [³H]-spiperone (70.3 Ci/mmol; 10-3000 pM final concentration) for 30-120 minutes at 22°C in a total volume of 1 ml. For competition binding experiments, assays are initiated by the addition of 0.75 ml of membrane and incubated in duplicate with the indicated concentrations of competing ligands (10⁻¹⁴-10⁻³ M) and/or [³H]-spiperone (100-300 pM) for 60-120 min at 22°C. Assays are terminated by rapid filtration through a Brandell cell harvester and the filters subsequently monitored for tritium as described by Sunahara, R.K., *et al.,* Nature, 346, 76-80 (1990). For all experiments, specific [³H]-spiperone binding is defined as that inhibited by 1-10 mM (+)-butaclamol. Binding data are analyzed by non-linear least squares curve-fitting. All of the compounds recited herein which were tested in this assay were found to have binding affinities (Kᵢ) for the displacement of [³H]-spiperone of less than 2 micromolar.

When administered in combination, either as a single or as separate pharmaceutical composition(s), the D4 receptor antagonist and the antidepressant or anti-anxiety agent, are presented in a ratio which is consistent with the manifestation of the desired effect. In particular, the ratio by weight of the D4 receptor antagonist and the antidepressant or anxiolytic agent will suitably be between 0.001 to 1 and 1000 to 1, and especially between 0.01 to I and 100 to 1.

## Claims

1. A pharmaceutical composition comprising: (a) a compound that exhibits activity, respectively, as an antidepressant or an anxiolytic agent, or a pharmaceutically acceptable salt thereof; (b) a D4 receptor antagonist or pharmaceutically acceptable salt thereof; and (c) a pharmaceutically acceptable carrier.

2. A pharmaceutical composition according to claim 1, wherein the D4 receptor antagonist or pharmaceutically acceptable salt thereof is selected from compounds of the formula I, as depicted and defined below, and their pharmaceutically acceptable salts: 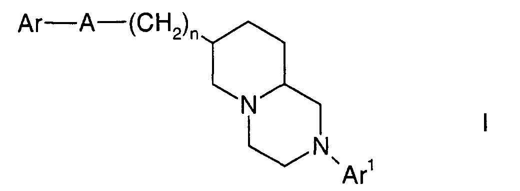
wherein Ar is phenyl, naphthyl, benzoxazolonyl, indolyl, indolonyl, benzimidazolyl, quinolyl, furyl, benzofuryl, thienyl, benzothienyl, oxazolyl or benzoxazolyl;
Ar¹ is phenyl, pyridinyl, pyridazinyl, pyrimidinyl or pyrazinyl;
A is O, S, SO, SO₂, C=O, CHOH or -(CR³R⁴)-;
n is 0, 1 or 2;
each of Ar and Ar¹ may be independently and optionally substituted with one to four substituents independently selected from the group consisting of fluoro, chloro, bromo, iodo, cyano, nitro, thiocyano, -SR, -SOR, -SO₂R, -NHSO₂R, -(C₁-C₆)alkoxy, -NR¹R², -NRCOR¹, -CONR¹R², phenyl, -COR, -COOR, -(C₁-C₆)alkyl, -(C₁-C₆)alkyl substituted with one to six halogens independently selected from fluoro, chloro, bromo and iodo, -(C₃-C₆)cycloalkyl and trifluoromethoxy;
each and every R, R¹, and R² is independently selected from the group consisting of hydrogen, -(C₁-C₆)alkyl, -(C₁-C₆)alkyl substituted with one to thirteen halogens independently selected from fluoro, chloro, bromo and iodo, phenyl, benzyl, -(C₂-C₆)alkenyl,
-(C₃-C₆)cycloalkyl and -(C₁-C₆)alkoxy; and
each and every R³ and R⁴ is independently selected from the group consisting of hydrogen, methyl, ethyl, n-propyl and i-propyl.

3. A pharmaceutical composition according to claim 1, wherein the D4 receptor antagonist or pharmaceutically acceptable salt thereof is selected from compounds of the formula II, as depicted and defined below, and their pharmaceutically acceptable salts: 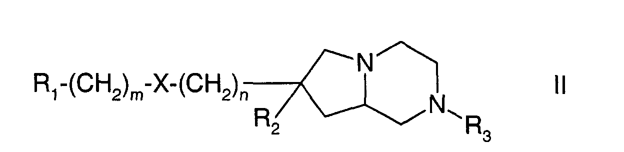
wherein R₁ is phenyl, naphthyl, benzoxazolonyl, indolyl, indolonyl, benzimidazolyl, quinolyl, furyl, benzofuryl, thienyl, benzothienyl, oxazolyl or benzoxazolyl;
R₂ is H or (C₁-C₆)alkyl;
R₃ is phenyl, pyridinyl, pyrimidinyl, pyrazinyl or pyridazinyl;
R₄ is H or (C₁-C₆)alkyl;
R₅ is H or (C₁-C₆)alkyl;
wherein each group of R₁ and R₃ may be independently and optionally substituted with one to four substituents independently selected from the groups consisting of fluoro, chloro, bromo, iodo, cyano, nitro, thiocyano, -SR₄, -SOR₄, -SO₂R₄, -NHSO₂R₄, -(C₁-C₆)alkoxy, -NR₄R₅, -NR₄COR₅, -CONR₄R₅, phenyl, -COR₄, -COOR₄, -(C₁-C₆)alkyl, -(C₁-C₆)alkyl substituted with one to six halogens, -(C₃-C₆)cycloalkyl and trifluoromethoxy;
X is O, S, SO, SO₂, NR₄, C=O, CH(OH), CHR₄, 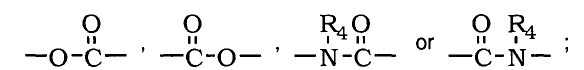
m is 0, 1 or 2; and
n is 0, 1 or 2.

4. A pharmaceutical composition according to claim 1, wherein the D4 receptor antagonist or pharmaceutically acceptable salt thereof is selected from compounds of the formula III or IIIA, as depicted and defined below, and their pharmaceutically acceptable salts: 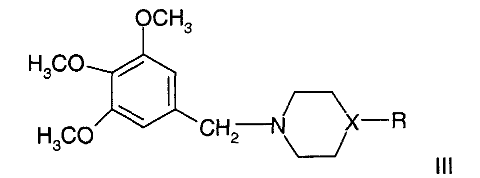
wherein X is N or CH; and
R is aryl or heteroaryl;
with the proviso that when X is N and R is aryl, aryl is not phenyl, phenyl monosubstituted by lower alkyl, lower alkoxy, halogen, or nitro, phenyl disubstituted by lower alkyl, or phenyl trisubstituted by lower alkoxy;
or 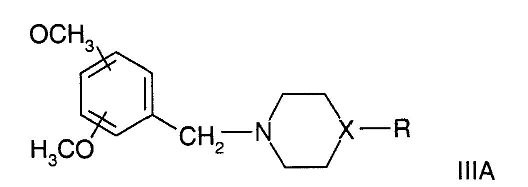
wherein X is N or CH; and
R is aryl or heteroaryl;
with the following provisos:
(a) that when X is N or CH, and R is aryl, aryl is not phenyl, or phenyl monosubstituted by lower alkyl, lower alkoxy, or halogen; and
(b) that when X is N and R is heteroaryl, heteroaryl is not 2-, 3-, or 4-pyridinyl.

5. A pharmaceutical composition according to claim 1, wherein the D4 receptor antagonist or pharmaceutically acceptable salt thereof is selected from compounds of the formula IV or IVA, as depicted and defined below, and their pharmaceutically acceptable salts: 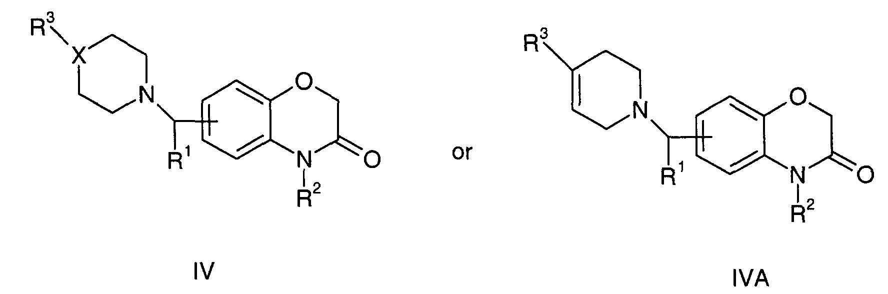 wherein
R¹ and R² are independently hydrogen or C₁-C₆ alkyl;
X is N or CH; and
R³ is phenyl, naphthyl, heteraryl, substituted phenyl, substituted naphthyl or substituted heteroaryl, wherein each substituent is independently selected from halogen, C₁-C6 alkoxy, C₁-C₆ alkyl, -CN, -CF₃ or sulphonamido.

6. A pharmaceutical composition according to claim 1, wherein the D4 receptor antagonist or pharmaceutically acceptable salt thereof is selected from compounds of the formula IVB, as depicted and defined below, and their pharmaceutically acceptable salts: 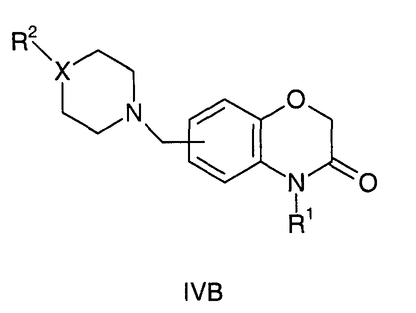 wherein
X is N or CH;
R¹ is hydrogen or methyl; and
R² is phenyl or substituted phenyl, wherein each substituent is independently selected from C₁-C₆ alkyl or sulphonamido.

7. A pharmaceutical composition according to any one of claims 1 to 6 wherein the amount of the antidepressant, or pharmaceutically acceptable salt thereof, in said composition is from 0.5 mg to 1500 mg per day or the amount of the anxiolytic agent, or pharmaceutically acceptable salt thereof, in said composition is 0.1 mg to 1500 mg per day, and the amount of the D4 receptor antagonist or pharmaceutically acceptable salt thereof is from 0.05 mg to 1500 mg per day.

8. A pharmaceutical composition according to claim 7 wherein the amount of the antidepressant, or pharmaceutically acceptable salt thereof, in said composition is from 2.5 mg to 750 mg per day or the amount of the anxiolytic agent, or pharmaceutically acceptable salt thereof, in said composition is 0.1 mg to 500 mg per day, and the amount of the D4 receptor antagonist or pharmaceutically acceptable salt thereof is from 5 mg to 500 mg per day.

9. Use of a pharmaceutical composition according to any one of claims 1 to 8 in the preparation of a medicament for the treatment of depression, anxiety or psychosis in a mammal.

10. A kit comprising: (a) a compound that exhibits activity as an antidepressant or an anxiolytic agent, or a pharmaceutically acceptable salt thereof; (b) a D4 receptor antagonist or pharmaceutically acceptable salt thereof; and (c) a container for (a) and (b).

11. A kit according to claim 10, wherein the D4 receptor antagonist or pharmaceutically acceptable salt thereof is selected from compounds of the formula I, as depicted and defined below 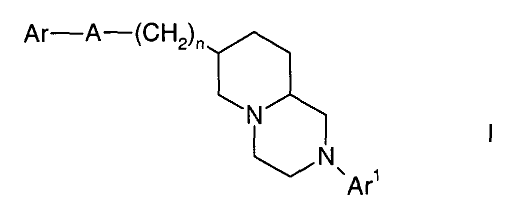
wherein Ar is phenyl, naphthyl, benzoxazolonyl, indolyl, indolonyl, benzimidazolyl, quinolyl, furyl, benzofuryl, thienyl, benzothienyl, oxazolyl or benzoxazolyl;
Ar¹ is phenyl, pyridinyl, pyridazinyl, pyrimidinyl or pyrazinyl;
A is O, S, SO, SO₂, C=O, CHOH or -(CR³R⁴)-;
n is 0, 1 or 2;
each of Ar and Ar¹ may be independently and optionally substituted with one to four substituents independently selected from the group consisting of fluoro, chloro, bromo, iodo, cyano, nitro, thiocyano, -SR, -SOR, -SO₂R, -NHSO₂R, -(C₁-C₆)alkoxy, -NR¹R², -NRCOR¹, -CONR¹R², phenyl, -COR, -COOR, -(C₁-C₆)alkyl, -(C₁-C₆)alkyl substituted with one to six halogens independently selected from fluoro, chloro, bromo and iodo, -(C₃-C₆)cycloalkyl and trifluoromethoxy;
each and every R, R¹, and R² is independently selected from the group consisting of hydrogen, -(C₁-C₆)alkyl, -(C₁-C₆)alkyl substituted with one to thirteen halogens independently selected from fluoro, chloro, bromo and iodo, phenyl, benzyl, -(C₂-C₆)alkenyl, -(C₃-C₆)cycloalkyl and -(C₁-C₆)alkoxy; and
each and every R³ and R⁴ is independently selected from the group consisting of hydrogen, methyl, ethyl, n-propyl and i-propyl;
compounds of the formula II, as depicted and defined below 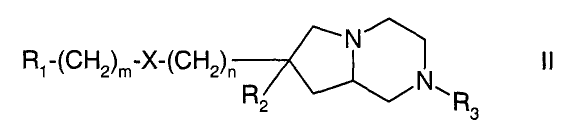
wherein R₁ is phenyl, naphthyl, benzoxazolonyl, indolyl, indolonyl, benzimidazolyl, quinolyl, furyl, benzofuryl, thienyl, benzothienyl, oxazolyl or benzoxazolyl;
R₂ is H or (C₁-C₆)alkyl;
R₃ is phenyl, pyridinyl, pyrimidinyl, pyrazinyl or pyridazinyl;
R₄ is H or (C₁-C₆)alkyl;
R₅ is H or (C₁-C₆)alkyl;
wherein each group of R₁ and R₃ may be independently and optionally substituted with one to four substituents independently selected from the groups consisting of fluoro, chloro, bromo, iodo, cyano, nitro, thiocyano, -SR₄, -SOR₄, -SO₂R₄, -NHSO₂R₄, -(C₁-C₆)alkoxy, -NR₄R₅, -NR₄COR₅, -CONR₄R₅, phenyl, -COR₄, -COOR₄, -(C₁-C₆)alkyl, -(C₁-C₆)alkyl substituted with one to six halogens, -(C₃-C₆)cycloalkyl and trifluoromethoxy;
X is O, S, SO, SO₂, NR₄, C=O, CH(OH), CHR₄, 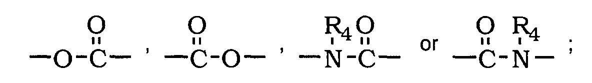
m is 0,1 or 2; and
n is 0, 1 or 2;
compounds of the formula III or IIIA, as depicted and defined below 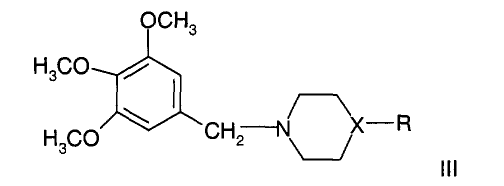
wherein X is N or CH; and
R is aryl or heteroaryl;
with the proviso that when X is N and R is aryl, aryl is not phenyl, phenyl monosubstituted by lower alkyl, lower alkoxy, halogen, or nitro, phenyl disubstituted by lower alkyl, or phenyl trisubstituted by lower alkoxy;
or 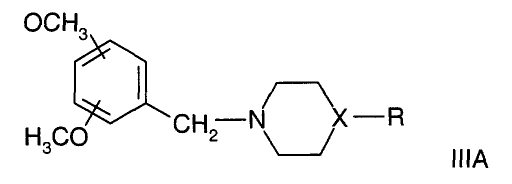
wherein X is N or CH; and
R is aryl or heteroaryl;
with the following provisos:
(a) that when X is N or CH, and R is aryl, aryl is not phenyl, or phenyl monosubstituted by lower alkyl, lower alkoxy, or halogen; and
(b) that when X is N and R is heteroaryl, heteroaryl is not 2-, 3-, or 4-pyridinyl;
compounds of the formula IV or IVA, as depicted and defined below 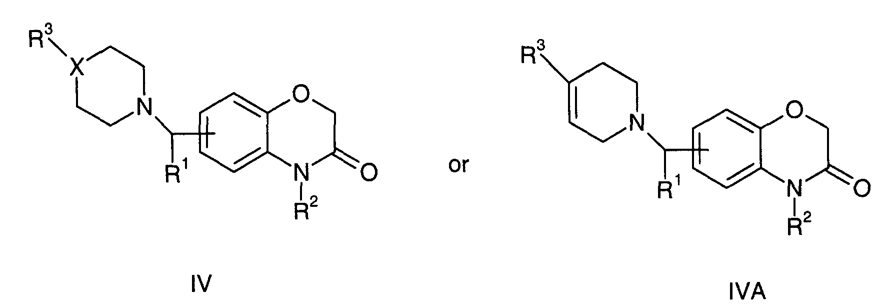
wherein R¹ and R² are independently hydrogen or C₁-C₆ alkyl;
X is N or CH; and
R³ is phenyl, naphthyl, heteraryl, substituted phenyl, substituted naphthyl or substituted heteroaryl, wherein each substituent is independently selected from halogen, C₁-C6 alkoxy, C₁-C₆ alkyl, -CN, -CF₃ or sulphonamido;
compounds of the formula IVB, as depicted and defined below 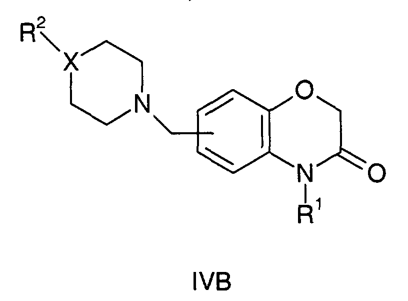
wherein X is N or CH;
R¹ is hydrogen or methyl; and
R² is phenyl or substituted phenyl, wherein each substituent is independently selected from C₁-C₆ alkyl or sulphonamido;
and their pharmaceutically acceptable salts.

12. A kit according to claim 11, wherein the D4 receptor antagonist or pharmaceutically acceptable salt thereof that is employed in such method is selected from the following compounds and their pharmaceutically acceptable salts:
(7R,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7R,9aS)-7-(3,5-difluorophenoxy)methyl-2-(5-chloro-pyridin-2-yl)-2,3,4,6,7,8,9,9a-octahydro-1*H*-pyrido[1,2-a]pyrazine;
3-[(7R,9aS)-2-(5-chloro-pyridin-2-yl)-2,3,4,6,7,8,9,9a-octahydro-1*H*-pyrido[1,2-a]pyrazin-7-ylmethyl]-3*H*-benzooxazol-2-one;
3-[(7R,9aS)-2-(5-fluoro-pyrimidin-2-yl)-2,3,4,6,7,8,9,9a-octahydro-1*H*-pyrido[1,2-a]pyrazin-7-ylmethyl]-3*H*-benzoxazol-2-one;
(7R,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,5-difluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,4-difluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-iodophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-fluorophenoxy)methyl-2-(4-fluorophenyl)-2,3,4,6,7,8,9,9a-octahydro-1*H*-pyrido[1,2-a]pyrazine;
(7S,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2-carbomethoxy-4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2-bromo-4-fluorophenoxy)methyl-2-(5-fluoro-pyrimidin-2-yl)-2,3,4,6,7,8,9,9a-octahydro-1 H-pyrido[1,2-a]pyrazine;
(7S,9aS)-7-(4-fluoro-2-trifluoromethylphenoxy)methyl-2-(5-fluoro-pyrimidin-2-yl)-2,3,4,6,7,8,9,9a-octahydro-1*H*-pyrido[1,2-a]pyrazine;
(7S,9aS)-7-(3,5-difluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluorophenoxy) methyl-2-(5-chloro-pyridin-2-yl)-2,3,4,6,7,8,9,9a-octahydro-1*H*-pyrido[1,2-a]pyrazine;
(7S,9aS)-7-(4-fluoro-2-methylphenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2,4-difluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(3-methyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,4-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,5-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-cyano-phenoxy)methyl-2-(5-fluoropyrimidin-2-yl)-2,3,4,6,7,8,9,9a-octahydro-1*H*-pyrido[1,2-a]pyrazine;
(75,9aS)-7-(3-trifluoromethyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(4-trifluoromethyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-trifluoromethoxy-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-methoxy-phenoxy)methyl-2-(5-fluoropyrimidin-2-yl)-2,3,4,6,7,8,9,9a-octahydro-1*H*-pyrido[1,2-a]pyrazine; and
(7S,9aS)-7-(4-methoxy-phenoxy)methyl-2-(5-fluoropyrimidin-2-yl)-2,3,4,6,7,8,9,9a-octahydro-1*H-*pyrido[1,2-a]pyrazine.

13. Use of a kit according to any one of claims 10 to 12 in the preparation of a medicament for treating depression, anxiety or psychosis in a mammal.

14. Use according to claim 13 wherein the antidepressant or anxiolytic agent, or pharmaceutically acceptable salt thereof, and the D4 receptor antagonist or pharmaceutically acceptable salt thereof, are administered as part of the same dosage form.

15. Use according to either claim 13 or claim 14 wherein the D4 receptor antagonist, or pharmaceutically acceptable salt thereof, is administered in an amount from 0.05 mg to 1500 mg per day, and the antidepressant, or pharmaceutically acceptable salt thereof, is administered in an amount from 0.5 mg to 1500 mg per day or the anxiolytic agent, or pharmaceutically acceptable salt thereof, is administered in an amount from 0.1 mg to 1500 mg per day.

16. Use according to claim 15 wherein the D4 receptor antagonist is administered in an amount ranging from 5 mg to 500 mg per day.

17. A pharmaceutical composition according to claim 2, wherein the D4 receptor antagonist or pharmaceutically acceptable salt thereof that is employed in such composition is selected from the following compounds and their pharmaceutically acceptable salts:
(7R,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7R,9aS)-7-(3,5-difluorophenoxy)methyl-2-(5-chloro-pyridin-2-yl)-2,3,4,6,7,8,9,9a-octahydro-1*H*-pyrido[1,2-a]pyrazine;
3-[(7R,9aS)-2-(5-chloro-pyridin-2-yl)-2,3,4,6,7,8,9,9a-octahydro-1*H*-pyrido[1,2-a]pyrazin-7-ylmethyl]-3*H*-benzooxazol-2-one;
3-[(7R,9aS)-2-(5-fluoro-pyrimidin-2-yl)-2,3,4,6,7,8,9,9a-octahydro-1*H*-pyrido[1,2-a]pyrazin-7-ylmethyl]-3*H*-benzoxazol-2-one;
(7R,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,5-difluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,4-difluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-iodophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-fluorophenoxy)methyl-2-(4-fluorophenyl)-2,3,4,6,7,8,9,9a-octahydro-1 *H*-pyrido[1,2-a]pyrazine;
(7S,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2-carbomethoxy-4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(2-bromo-4-fluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(4-fluoro-2-trifluoromethylphenoxy)methyl-2-(5-fluoro-pyrimidin-2-yl)-2,3,4,6,7,8,9,9a-octahydro-1*H*-pyrido[1,2-a]pyrazine;
(7S,9aS)-7-(3,5-difluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluoro-2-methylphenoxy)methyl-2-(5-fluoro-pyrimidin-2-yl)-2,3,4,6,7,8,9,9a-octahydro-1*H-*pyrido[1,2-a]pyrazine;
(7S,9aS)-7-(2,4-difluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(3-methyl-phenoxy)methyl-2-(5-fluoropyrimidin-2-yl)-2,3,4,6,7,8,9,9a-octahydro-1*H-*pyrido[1,2-a]pyrazine;
(7S,9aS)-7-(3,4-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,5-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-cyano-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-trifluoromethyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(4-trifluoromethyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-trifluoromethoxy-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-methoxy-phenoxy)methyl-2-(5-fluoropyrimidin-2-yl)-2,3,4,6,7,8,9,9a-octahydro-1*H*-pyrido[1,2-a]pyrazine; and
(7S,9aS)-7-(4-methoxy-phenoxy)methyl-2-(5-fluoropyrimidin-2-yl)-2,3,4,6,7,8,9,9a-octahydro-1*H*-pyrido[1,2-a]pyrazine.

18. A product comprising (a) a compound that exhibits activity as an antidepressant or an anxiolytic agent, or a pharmaceutically acceptable salt thereof, and (b) a D4 receptor antagonist or pharmaceutically acceptable salt thereof as a combined preparation for simultaneous, separate or sequential use in treating depression, anxiety or psychosis in a mammal

19. A pharmaceutical composition according to claim 1 for use in medicine.
